(19) Europäisches Patentamt
European Patent Office
Office européen des brevets

(11) Veröffentlichungsnummer: **0 417 630 A2**

# (12) EUROPÄISCHE PATENTANMELDUNG

(21) Anmeldenummer: **90117077.9**

(22) Anmeldetag: **05.09.90**

(51) Int. Cl.[5]: **C07D 519/00**, C07D 487/08, A61K 31/55, //(C07D519/00, 487:00,471:00),(C07D519/00, 495:00,487:00),(C07D519/00, 471:00,471:00),(C07D519/00, 487:00,487:00),(C07D487/08, 209:00,209:00)

(30) Priorität: **11.09.89 DE 3930266**

(43) Veröffentlichungstag der Anmeldung:
**20.03.91 Patentblatt 91/12**

(84) Benannte Vertragsstaaten:
**AT BE CH DE DK ES FR GB GR IT LI LU NL SE**

(71) Anmelder: **Dr. Karl Thomae GmbH**
**Postfach 1755**
**W-7950 Biberach 1(DE)**

(72) Erfinder: **Rudolf, Klaus, Dr.**
**Marktplatz 38**
**W-7950 Biberach 1(DE)**
Erfinder: **Engel, Wolfhard, Dr. Dipl.-Chem.**
**Mozartstrasse 13**
**W-7950 Biberach 1(DE)**
Erfinder: **Eberlein, Wolfgang, Dr. Dipl.-Chem.**
**Obere Au 6**
**W-7950 Biberach 1(DE)**
Erfinder: **Trummlitz, Günter, Dr. Dipl.-Chem.**
**Buchenweg 27**
**W-7951 Warthausen(DE)**
Erfinder: **Mihm, Gerhard, Dr. Dipl.-Chem.**
**Nickeleshalde 5/1**
**W-7950 Biberach 1(DE)**
Erfinder: **Doods, Henri, Dr.**
**Hornsteinweg 7**
**W-7951 Warthausen(DE)**
Erfinder: **Mayer, Norbert, Dr.**
**Friedrich-Ebert-Strasse 66**
**W-7950 Biberach 1(DE)**

(54) **Kondensierte Diazepinone, Verfahren zu ihrer Herstellung und diese Verbindungen enthaltende Arzneimittel.**

(57) Kondensierte Diazepinone der allgemeinen Formel I

EP 0 417 630 A2

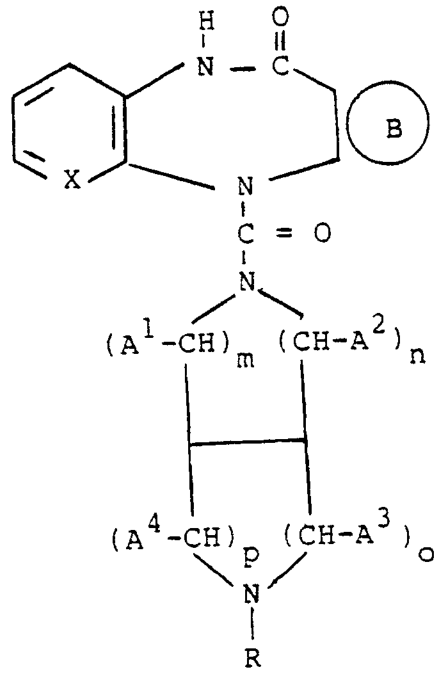

(I)

in der

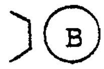

einen der zweiwertigen Reste

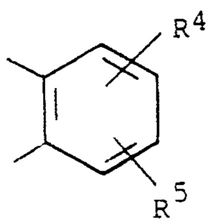 , 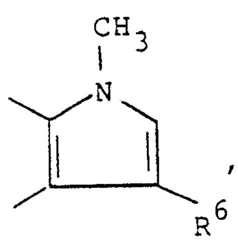 , 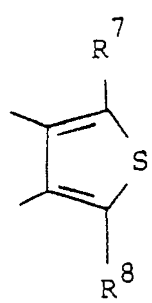 , 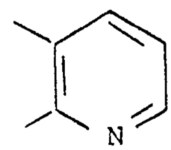

(S) (T) (U). (V)

darstellt, X eine =CH-Gruppe oder ein Stickstoffatom ist, R einen niederen Alkylrest der gegebenenfalls noch substituiert sein kann durch ein gegebenenfalls Halogen, Methyl oder Methoxy tragendes Phenyl, $R^4$ und $R^5$ Wasserstoff, Halogen oder niederes Alkyl darstellen, $R^6$ Wasserstoff, Chlor oder Methyl ist, $R^7$ und $R^8$ niederes Alkyl, $R^8$ zusätzlich noch Halogen bedeuten und m, n, o und p die Zahlen 0, 1, 2 oder 3 mit folgenden Einschränkungen darstellen: die Summe aus m+n und die Summe aus o+p bedeutet jeweils die Zahlen 1, 2 oder 3, die Summe aus n+o und die Summe aus m+p jeweils die Zahlen 1, 2, 3, 4 oder 5, wobei jedoch die Summe aus m+n+o+p immer größer als 2 sein muß, und $A^1$, $A^2$, $A^3$, $A^4$ Wasserstoff oder, für den Fall, daß m, n, o und p jeweils die Zahl 1 bedeuten, $A^1$ und $A^2$ zusammen oder $A^3$ und $A^4$ zusammen eine Ethylenbrücke darstellen, eignen sich zur Therapie cholinerg bedingter Spasmen und Motilitätsstörungen im Magen-Darm-Trakt, im Bereich der abführenden Gallengänge, zur symptomatischen Therapie der Cystitis und von Spasmen bei Urelithiasis, zur Therapie der relativen Inkontinenz, zur symptomatischen Therapie des Asthma bronchiale und der Bronchitis sowie zur Therapie ischämischer Herzerkrankungen. Die Verbindungen zeichnen sich durch eine gute Selektivität aus.

## KONDENSIERTE DIAZEPINONE, VERFAHREN ZU IHRER HERSTELLUNG UND DIESE VERBINDUNGEN ENTHALTENDE ARZNEIMITTEL

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424. 225; 4.424.222 und 4.424.226 sind bereits kondensierte Diazepinone mit ulcushemmenden und magensaftsekretionshemmenden Eigenschaften bekannt.

In EP-A-0 156 191 (US-Patent 4 550 107) ist für kondensierte Diazepinone beschrieben, daß durch Einführung neuartiger Aminoacylreste gegenüber den Verbindungen der obengenannten Publikationen völlig andersartige, wertvolle pharmakologische Eigenschaften induziert werden können. Gegenüber den oben genannten ulcushemmenden und den vorstehend erwähnten antibradycarden kondensierten Diazepinonen weisen die Verbindungen der vorliegenden Anmeldung - trotz naher struktureller Verwandschaft - überraschend eine weitere, von den obigen Effekten abweichende Wirkqualität auf. Die erfindungsgemäßen Verbindungen eignen sich zur Therapie cholinerg bedingter Spasmen und Motilitätsstörungen im Magen-Darm-Trakt und im Bereich der abführenden Gallengänge, zur symptomati schen Therapie der Cystitis und von Spasmen bei Urelithiasis durch Senkung des pathologisch erhöhten Tonus der Hohlorgane, zur Therapie der relativen Inkontinenz, die auf einem Mißverhältnis von Sphinkter- und Detrusor-Tonus beruht, zur symptomatischen Therapie des Asthma bronchiale und der Bronchitis durch Unterdrückung des muscarinisch vermittelten Anteils der Bronchokonstriktion, sowie zur Therapie ischämischer Herzerkrankungen durch Herzfrequenzsenkung bei gleichzeitiger Unterdrückung parasympathisch verursachter Coronarspasmen und Senkung des basalen Coronartonus. Die erfindungsgemäßen kondensierten Diazepinone zeigen die angegebenen Effekte mit hoher Selektivität und sind insbesondere im therapeutisch relevanten Dosisbereich frei von tachycarden Begleitwirkungen.

Die neuen kondensierten Diazepinone besitzen die allgemeine Formel I

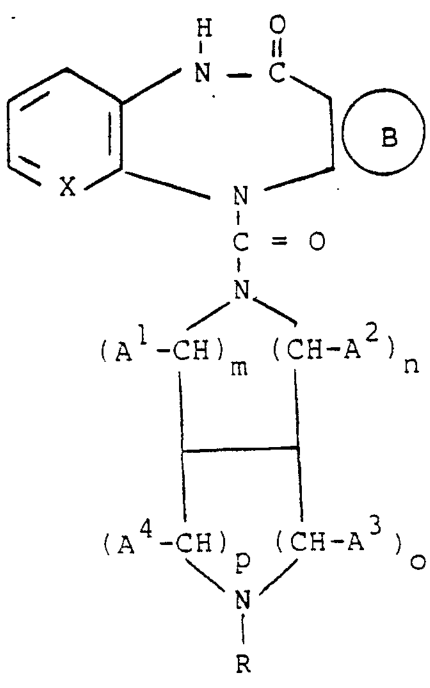

(I)

in der

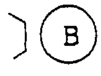

einen der zweiwertigen Reste

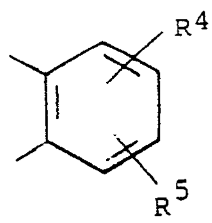 , 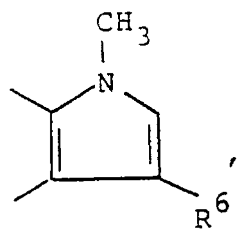 , 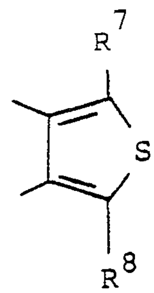 , 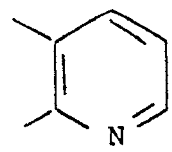

(S) (T) (U) (V)

darstellt und

X, R, $R^4$, $R^5$, $R^6$, $R^7$, $R^8$, m, n, o, p und $A^1$, $A^2$, $A^3$ und $A^4$ die folgenden Bedeutungen besitzen:

X ist eine =CH-Gruppe oder ein Stickstoffatom,

R ist ein geradkettiger oder verzweigter Alkylrest mit 1 bis 4 Kohlenstoffatomen, der gegebenenfalls noch durch ein durch Chlor, Brom, Fluor, Methyl oder Methoxy mono- oder disubstituiertes Phenyl substituiert sein kann,

$R^4$ und $R^8$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^5$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 0, 1, 2 oder 3 mit folgenden Einschränkungen:

die Summe aus m+n und die Summe aus o+p bedeutet jeweils die Zahlen 1, 2 oder 3,

die Summe aus n+o und die Summe aus m+p jeweils die Zahlen 1, 2, 3, 4 oder 5,

wobei jedoch die Summe aus m+n+o+p immer größer als 2 sein muß,

$A^1$, $A^2$, $A^3$, $A^4$ bedeuten Wasserstoffatome; für den Fall, daß m, n, o und p jeweils die Zahl 1 bedeuten, können entweder $A^1$ und $A^2$ zusammen oder $A^3$ und $A^4$ zusammen auch eine Ethylenbrücke darstellen.

Zur Erläuterung des Restes der allgemeinen Formel

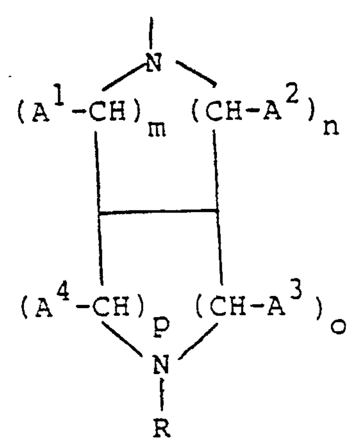

seinen folgende Strukturen erwähnt:

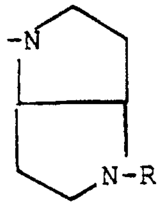 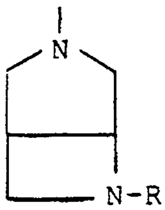 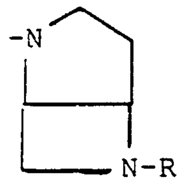 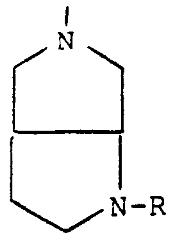

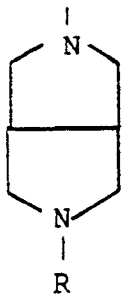 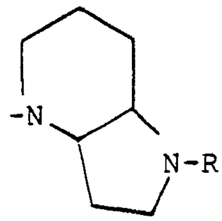 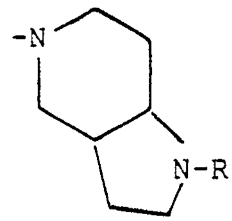 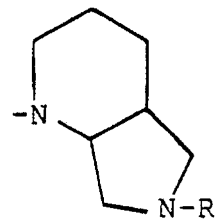

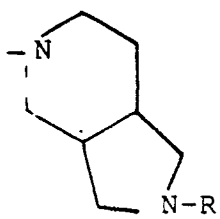 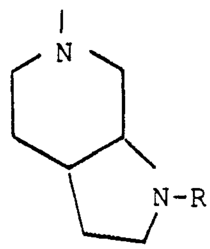 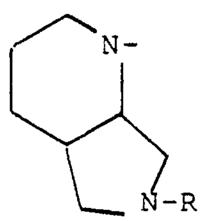 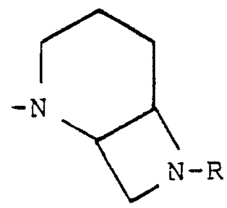

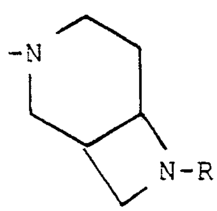 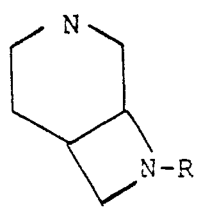 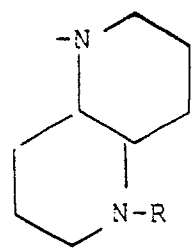 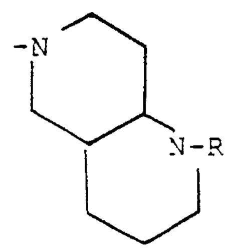

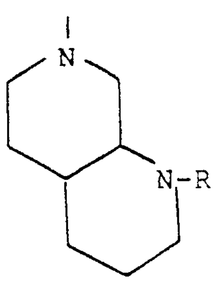 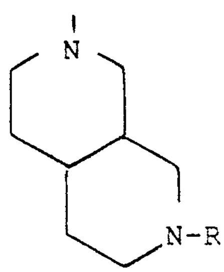 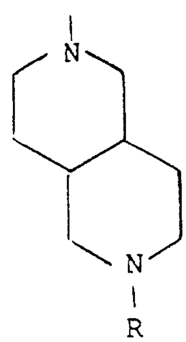 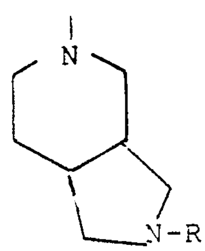 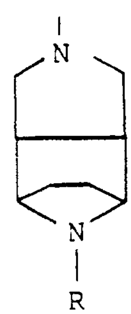 ,

wobei in den einzelnen Ringsystemen die Bindung zur Carbonylgruppe am Tricyclus des einen Stickstoffatoms auch gegen den Rest R des anderen Stickstoffatoms ausgetauscht sein kann, die auch, sofern sie die später genannten Kriterien erfüllen, als cis- oder trans-Isomere vorliegen können.

Bevorzugte Verbindungen der obigen allgemeinen Formel I sind solche, in denen entweder

X ein Stickstoffatom und

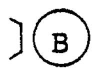

den Rest (S) oder
X die =CH-Gruppe und

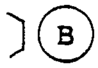

den Rest (V) bedeuten,
R die Methylgruppe, und
$R^4$ und $R^5$, die gleich oder voneinander verschieden sein können, jeweils ein Wasserstoff-, Fluor- oder Chloratom, die Methyl- oder Ethylgruppe darstellen und
m = 0, n = 2, o = 0, p = 2 oder m, n, o und p jeweils gleich 1 sind.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:
5,11-Dihydro-11-[[7-methyl-3,7-diazabicyclo[3,3,0]oct-3-yl]-carbonyl]-6H-pyrido[2,3-b][1,4]benzodiazepin-6-on
9-Chlor-5,11-dihydro-11-[[7-methyl-3,7-diazabicyclo[3,3,0]oct-3-yl]carbonyl]-6H-pyrido[2,3-b][1,4]-benzodiazepin-6-on
6,11-Dihydro-11-[[7-methyl-3,7-diazabicyclo[3.3.0]oct-3-yl]carbonyl]-5H-pyrido[2,3-b][1,5]benzodiazepin-5-on
L-5,11-Dihydro-11-[[6-methyl-2,6-diazabicyclo[3,3,0]oct2-yl]carbonyl]-6H-pyrido[2,3-b][1,4]benzodiazepin-6-on
L-5,11-Dihydro-8-methyl-11-[[6-methyl-2,6-diazabicyclo[3,3,0]oct-2-yl]carbonyl]-6H-pyrido[2,3-b][1,4]-benzodiazepin-6-on
L-6,11-Dihydro-11-[[6-methyl-2,6-diazabicyclo[3,3,0]oct-2-yl]carbonyl]-5H-pyrido[2,3-b][1,5]benzodiazepin-5-on
D-5,11-Dihydro-11-[[6-methyl-2,6-diazabicyclo[3,3,0]oct-2-yl]carbonyl]-6H-pyrido[2,3-b][1,4]benzodiazepin-6-on
D-5,11-Dihydro-8-ethyl-11-[[6-methyl-2,6-diazabicyclo[3,3,0]oct-2-yl]carbonyl]-6H-pyrido[2,3-b][1,4]-benzodiazepin-6-on
D-6,11-Dihydro-11-[[6-methyl-2,6-diazabicyclo[3,3,0]oct-2-yl]carbonyl]-5H-pyrido[2,3-b][1,5]benzodiazepin-5-on
L-5,11-Dihydro-11-[[6-isopropyl-2,6-diazabicyclo[3.3.0]oct-2-yl]carbonyl]-6H-pyrido[2,3-b][1,4]benzodiazepin-6-on
L-5,11-Dihydro-11-[[6-isobutyl-2,6-diazabicyclo[3.3.0]oct-2-yl]carbonyl]-6H-pyrido[2,3-b][1,4]benzodiazepin-6-on
cis-5,11-Dihydro-11-[[8-methyl-2,8-diazabicyclo[4.4.0]dec-2-yl]carbonyl]-6H-pyrido[2,3-b][1,4]benzodiazepin-6-on
trans-5,11-Dihydro-11-[[8-methyl-2,8-diazabicyclo[4.4.0]dec-2-yl]carbonyl]-6H-pyrido[2,3-b][1,4]-benzodiazepin-6-on
5,11-Dihydro-11-[[10-methyl-4,10-diazatricyclo[5.2.1.$0^{2,6}$]dec-4-yl]carbonyl]-6H-pyrido[2,3-b][1,4]-benzodiazepin-6-on 5,11-Dihydro-11-[[10-methyl-4,10-diazatricyclo[5.2.1.$0^{2,6}$]dec-4-yl]carbonyl]-6H-dibenzo-[2,3-b][1,4]diazepin-6-on
5,11-Dihydro-11-[[2-methyl-2,7-diazabicyclo[3.3.0]oct-7-yl]carbonyl]-6H-pyrido[2,3-b][1,4]benzodiazepin-6-on
6,11-Dihydro-11-[[2-methyl-2,7-diazabicyclo[3.3.0]oct-7-yl]carbonyl]-5H-pyrido[2,3-b][1,5]benzodiazepin-5-on
5,11-Dihydro-11-[[7-methyl-2,7-diazabicyclo[3.3.0]oct-2-yl]carbonyl]-6H-pyrido[2,3-b][1,4]benzodiazepin-6-on
6,11-Dihydro-11-[[7-methyl-2,7-diazabicyclo[3.3.0]oct-2-yl]carbonyl]-5H-pyrido[2,3-b][1,5]benzodiazepin-5-on
6,11-Dihydro-11-[[3-methyl-3,6-diazabicyclo[3.2.0]hept-6-yl]carbonyl]-5H-pyrido[2,3-b][1,5]benzodiazepin-5-on
5,11-Dihydro-11-[[3-methyl-3,6-diazabicyclo[3.2.0]hept-6-yl]carbonyl]-6H-pyrido[2,3-b][1,4]benzodiazepin-6-

on
5,11-Dihydro-11-[[6-methyl-3,6-diazabicyclo[3.2.0]hept-3-yl]carbonyl]-6H-pyrido[2,3-b][1,4]benzodiazepin-6-on
6,11-Dihydro-11-[[6-methyl-3,6-diazabicyclo[3.2.0]hept-3-yl]carbonyl]-5H-pyrido[2,3-b][1,5]benzodiazepin-5-on
5,11-Dihydro-11-[[3-methyl-3,8-diazabicyclo[4.2.0]oct-8-yl]carbonyl]-6H-pyrido[2,3-b][1,4]benzodiazepin-6-on
trans-5,11-Dihydro-11-[[7-methyl-2,7-diazabicyclo[4.4.0]dec-2-yl]-carbonyl]-6H-pyrido[2,3-b][1,4]-benzodiazepin-6-on
trans-6,11-Dihydro-11-[[7-methyl-2,7-diazabicyclo[4.4.0]dec-2-yl]-carbonyl]-5H-pyrido[2,3-b][1,5]-benzodiazepin-5-on
5,11-Dihydro-11-[[8-methyl-3,8-diazabicyclo[4.2.0]oct-3-yl]carbonyl]-6H-pyrido[2,3-b][1,4]benzodiazepin-6-on
5,11-Dihydro-11-[[3-methyl-3,7-diazabicyclo[4.2.0]oct-7-yl]carbonyl]-6H-pyrido[2,3-b][1,4]benzodiazepin-6-on
5,11-Dihydro-11-[[7-methyl-3,7-diazabicyclo[4.2.0]oct-3-yl]carbonyl]-6H-pyrido[2,3-b][1,4]benzodiazepin-6-on
5,11-Dihydro-11-[[3-methyl-3,8-diazabicyclo[4.3.0]non-8-yl]carbonyl]-6H-pyrido[2,3-b][1,4]benzodiazepin-6-on
5,11-Dihydro-11-[[8-methyl-3,8-diazabicyclo[4.3.0]non-3-yl]carbonyl]-6H-pyrido[2,3-b][1,4]benzodiazepin-6-on
L-4,9-Dihydro-3-methyl-4-[[6-methyl-2,6-diazabicyclo[3.3.0]oct-2-yl]carbonyl]-10H-thieno[3,4-b][1,5]-benzodiazepin-10-on
4,9-Dihydro-3-methyl-4-[[7-methyl-3,7-diazabicyclo[3.3.0]oct-3-yl]carbonyl]-10H-thieno[3,4-b][1,5]-benzodiazepin-10-on
L-3-Chlor-1-methyl-4-[[6-methyl-2,6-diazabicyclo[3.3.0]oct-2-yl]carbonyl]-1,4,9,10-tetrahydropyrrolo[3,2-b]-[1,5]benzodiazepin-10-on
3-Chlor-1-methyl-4-[[7-methyl-3,7-diazabicyclo[3.3.0]oct-3-yl]carbonyl]-1,4,9,10-tetrahydropyrrolo[3,2-b][1,5]-benzodiazepin-10-on

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia

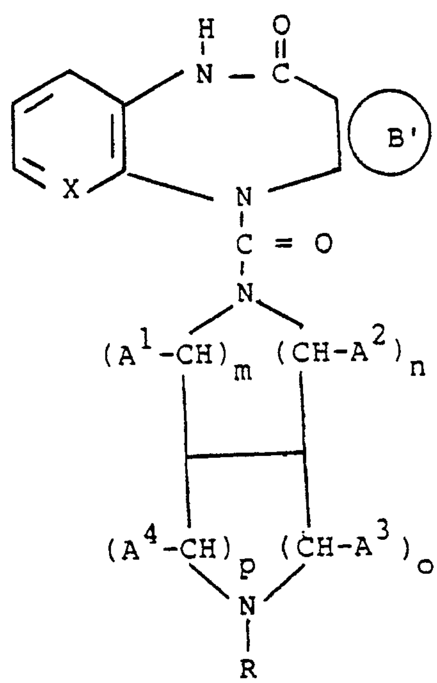

(Ia)

in der
X, R, $R^4$, $R^5$, $R^6$, $R^7$, $R^8$, m, n, o, p, $A^1$, $A^2$, $A^3$, $A^4$
die oben angegebenen Bedeutungen haben und

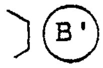

einen der zweiwertigen Reste (S), (U), (V) oder (T′)

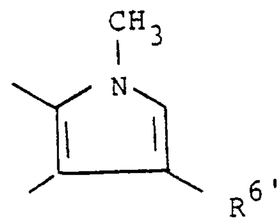
(T')

darstellt, wobei $R^{6'}$ ein Chloratom oder eine Methylgruppe ist,
erhält man durch Umsetzung von Kohlensäurederivaten der allgemeinen Formel II

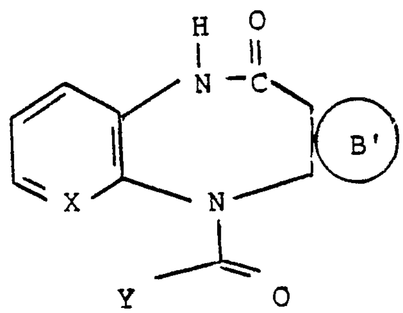
(II)

in der

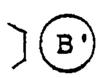

und X die angegebenen Bedeutungen haben und
Y ein Halogenatom, bevorzugt das Brom- oder Chloratom, oder den $OR^{11}$ est bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

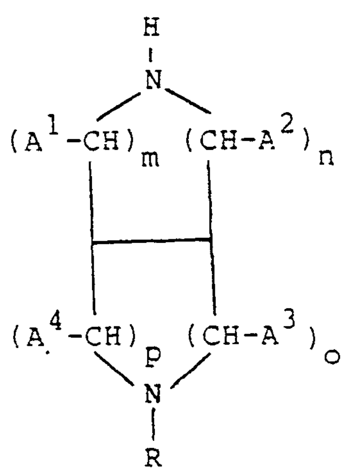
(III)

in der
R, m, n, o, p, $A^1$, $A^2$, $A^3$, $A^4$ wie vorstehend definiert sind.

Die Umsetzung wird ohne oder bevorzugt in Gegenwart von Lösungsmitteln, wie z. B. von Wasser, Toluol oder von Alkoholen, wie z. B Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen -10 ° C und dem Siedepunkt des Reaktionsgemisches, bevorzugt zwischen 40 und 100 ° C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat; von tertiären Aminen, z. B. von Triethylamin, Ethyldiisopropylamin, N,N-Dimethylanilin, Pyridin oder von 4-(Dimethylamino)pyridin; sowie die Umsetzung in Gegenwart eines Überschusses einer Verbindung der allgemeinen Formel III.

Verwendet man die bicyclischen Diamine der allgemeinen Formel III und die Kohlensäurederivate der allgemeinen Formel II in äquimolaren Mengenverhältnissen, so erhält man - sofern Y ein Halogenatom bedeutet - direkt die halogenwasserstoffsauren Salze der gesuchten Verbindungen der allgemeinen Formel Ia.

Die Umsetzung kann jedoch auch mit einer Metallverbindung der allgemeinen Formel IIIa,

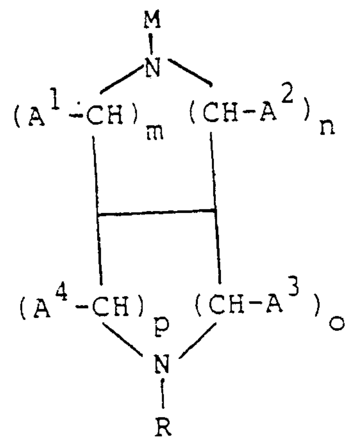

(IIIa)

in der
M ein Alkalimetallatom oder 1 Äquivalent eines Erdalkalimetallatoms bedeutet, durchgeführt werden. Dabei lassen sich Metallverbindungen der allgemeinen Formel IIIa aus III durch Umsetzung mit Alkali- oder Erdalkalimetallen, etwa mit Natrium, Kalium oder Barium, oder mit Alkali-.oder Erdalkalihydriden, etwa mit Natrium-, Kalium- oder Calciumhydrid, oder durch Reaktion mit Alkali- oder Erdalkaliorganylen, z. B. mit n-Butyllithium oder Phenyllithium, in situ leicht herstellen.

b.) Basisch substituierte kondensierte Diazepinone der allgemeinen Formel Ia lassen sich auch durch Umsetzung von Tricyclen der allgemeinen Formel IV,

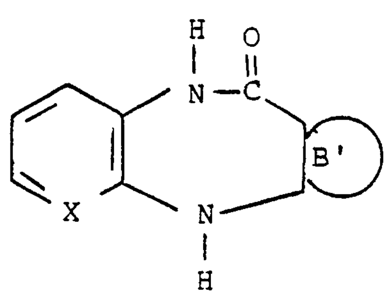

(IV)

in der X und

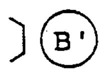

wie oben definiert sind, mit einem Chlorkohlensäurederivat der allgemeinen Formel V

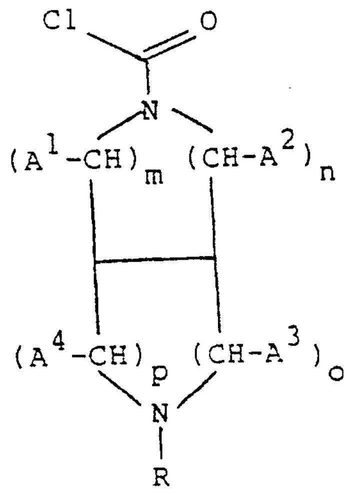

, (V)

worin R, m, n, o, p, $A^1$, $A^2$, $A^3$, $A^4$ die oben erwähnten Bedeutungen haben, erhalten.

Die Umsetzung wird vorzugsweise in inerten organischen Lösungsmitteln, beispielsweise in aromatischen Kohlenwasserstoffen wie Toluol, Xylol, in Ethern wie Diisopropylether, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen +30 und +100 °C, durchgeführt.

c) Die unter die allgemeine Formel I fallenden neuen Pyrrolokondensierten Diazepinone der allgemeinen Formel Ib,

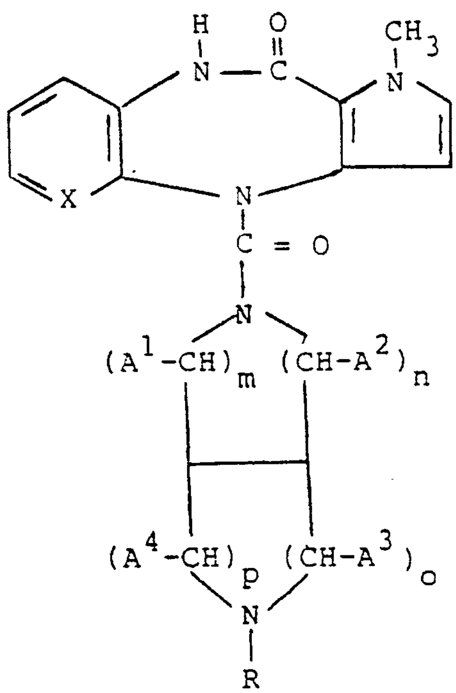

(Ib)

worin

X, R, m, n, o, p, $A^1$, $A^2$, $A^3$ und $A^4$ die eingangs erwähnten Bedeutungen haben, können auch durch Hydrogenolyse aus solchen Verbindungen der allgemeinen Formel Ia hergestellt werden, in denen $R^{5'}$ ein Chloratom bedeutet.

Die Hydrogenolyse wird in Gegenwart von Katalysatoren von Metallen aus der VIII. Nebengruppe des

Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0° C bis 130° C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110° C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110° C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Besitzen in den erfindungsgemäßen aminocarbonylierten kondensierten Diazepinonen der allgemeinen Formel I m, n, o oder p jeweils den Wert 1, so sind diese Verbindungen achiral, in allen anderen Fällen sind die Verbindungen jedoch chiral. Diese chiralen Verbindungen können deshalb jeweils als enantiomere (+)- und (-)-Formen auftreten. Die Erfindung umfaßt die einzelnen Isomeren ebenso wie ihre Racemate.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit. getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumhydroxid oder Kaliumhydroxid, neutralisiert und dadurch die entsprechende freie Verbindung in der (+)- oder (-)-Form erhalten.

Jeweils nur ein Enantiomeres wird auch dadurch erhalten, daß man die oben beschriebenen Synthesen mit nur einem Enantiomeren der allgemeinen Formel III bzw. V durchführt.

Die Herstellung der als Zwischenprodukte benötigten Kohlensäurederivate der allgemeinen Formel II wird in der DE-37 26 908-A1 ausführlich beschrieben.

Zwischenverbindungen der allgemeinen Formel III, die zum Teil neu und noch nicht vorbeschrieben sind, sind beispielsweise nach folgenden Methoden zugänglich:

a.) Die unter die allgemeine Formel III fallenden 2-substituierten 2,6-Diazabicyclo[3.3.0]octane erhält man entsprechend oder in völliger Analogie zu den. Angaben von Cope, A.C. und Shen, T.Y. in J. Am. Chem. Soc. 1956 , 78, 5916.

Das in dieser Literaturstelle vorbeschriebene 2-Benzyl-2,6-diazabicyclo[3.3.0]octan wird anschließend mit Chlorameisensäureethylester zum entsprechenden Urethan umgesetzt, welches dann mit Lithiumaluminiumhydrid zum 2-Benzyl-6-methyl-2,6-diazabicyclo[3.3.0]octan reduziert wird. Die hydrogenolytische Entfernung des Benzylrestes ergibt das gewünschte 2-Methyl-2,6-diazabicyclo[3.3.0]octan.

b.) Die unter die allgemeine Formel III fallenden 3-substituierten 3,7-Diazabicyclo[3.3.0]octane lassen sich beispielsweise ausgehend von Pyrrol-3,4-dicarbonsäure in völliger Analogie zu den Angaben von Loftus, P. und Wong, J.J. in J. Heterocyclic. Chem. 1983 , 20, 321 herstellen.

Alternativ können ausgehend von N-alkylierten Glycinderivaten, Paraformaldehyd und Maleinsäureimid und anschließender Reduktion des in dieser [3+2] Cycloaddition entstandenen bicyclischen Imids mit Lithiumaluminiumhydrid die gewünschten 3-substituierten 3,7-Diazabicyclo[3.3.0]octane hergestellt werden.

c.) Die unter die allgemeine Formel III fallenden 2-substituierten 2,7-Diaza-bicyclo[3.3.0]octane sind nach den Angaben von Birkhofer L. und Feldmann H. in Annalen 1964 , 677, 154 zugänglich.

d.) Das unter die allgemeine Formel III fallende 10-Methyl-4,10-diazatricyclo[$5.2.1.0^{2,6}$] decan ist beispielsweise aus dem literaturbekannten (Bansal, R.C. et.al., Can. J. Chem. 1969 , 47, 2391-4) N-Ethoxycarbonyl-7-azabicyclo[2.2.1]hepta-2,5-dien-2,3-dicarbonsäuredimethylester erhältlich. Alkalische Verseifung beider Methylestergruppen gefolgt von einer Palladium-katalysierten Hydrierung ergibt N-Ethoxycarbonyl-7-azabicyclo[2.2.1]heptan-2,3-dicarbonsäure in sehr guter Ausbeute. Die Dicarbonsäure wird mit Hilfe von Dicyclohexylcarbodiimid zum Anhydrid cyclisiert und dieses anschließend mit überschüssigem Benzylamin unter Rückfluß erhitzt. Das so in guter Ausbeute erhaltene 4-Benzyl-10-ethoxycarbonyl-4,10-diazatricyclo[5.2.1.$0^{2,6}$] decan-3,5-dion wird mit Lithiumaluminiumhydrid reduziert und die Benzylschutzgruppe schließlich durch katalytisch erregten Wasserstoff abgespalten.

e.) Die unter die allgemeine Formel III fallenden 8-substituierten 2,8-Diazabicyclo[4.4.0]decane können beispielsweise ausgehend von N-substituierten 4-Piperidonen erhalten werden. Das durch Reaktion mit z. B. Pyrrolidin erhaltene Enamin wird zunächst an Acrylnitril addiert und das Additionsprodukt in Schwefelsäure zu 8-substituierten $\Delta^{1,6}$-2,8-Diazabicyclo[4.4.0]decen-3-one cyclisiert. Hydrierung der Doppelbindung mit katalytisch erregtem Wasserstoff ergibt bevorzugt cis-verknüpfte, Hydrierung mit Triethylsilan jedoch bevorzugt trans-verknüpfte Ringsysteme. Reduktion der so erhaltenen 8-substituierten 2,8-Diazabicyclo-[4.4.0]decan-3-one zu den gewünschten 8-substituierten 2,8-Diazabicyclo[4.4.0]decanen gelingt glatt mit Lithiumaluminiumhydrid.

f.) Das unter die allgemeine Formel III fallende trans-7-Methyl-2,7-diazabicyclo[4.4.0]decan kann beispielsweise ausgehend vom 1,5-Naphthyridin hergestellt werden. Umsetzung mit Natrium in Amylalkohol ergibt das trans-2,7-Diazabicyclo[4.4.0]decan welches mit Chlorameisensäuremethylester zum Monourethan umgesetzt (Arch. Immunol. Ther. Exp. 1971 , 19, 261) wird. Behandlung mit Lithiumaluminiumhydrid ergibt das gewünschte trans-7-Methyl-2,7-diazabicyclo[4.4.0]decan. Alternativ kann das trans-2,7-Diazabicyclo-[4.4.0]decan mit Formaldehyd und Wasserstoff unter Zusatz eines Katalysators direkt in das trans-7-Methyl-2,7-diazabicyclo[4.4.0]decan umgesetzt werden.

Ein weiterer Gegenstand der Erfindung sind Arzneimittel, die ein oder mehrere basisch substituierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt bei oraler Gabe im allgemeinen zwischen 0,01 und 10 mg/kg, vorzugsweise 0,02 und 5 mg/kg, insbesondere 0,05 und 2,5 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; wie bereits eingangs ausgeführt, zeigen sie selektive spasmolytische Eigenschaften auf periphere Organe, insbesondere Ileum und Blase, und sind angesichts im therapeutischen Dosisbereich fehlender herzfrequenzsteigernder, magensäuresekretionshemmender, salivationshemmender und das Akkomodationsvermögen des Auges beeinträchtigender Effekte in der Human- und Veterinärmedizin geeignet zur Therapie cholinerg bedingter Spasmen und Motilitätsstörungen im Magen- Darm-Trakt und im Bereich der abführenden Gallengänge, zur symptomatischen Therapie der Cystitis und von Spasmen bei Urelithiasis durch Senkung des pathologisch erhöhten Tonus der Hohlorgane, zur Therapie der relativen Inkontinenz, die auf einem Mißverhältnis von Sphinkter- und Detrusor-Tonus beruht, zur sympthomatischen Therapie des Asthma bonchiale und der Bronchitis durch Unterdrückung des muscarinisch ermittelten Anteils der Bronchokonstriktion, sowie zur Therapie ischämischer Herzerkrankungen durch Herzfrequenzsenkung und gleichzeitiger Unterdrückung parasympathisch verursachter Coronarspasmen und Senkung des basalen Coronartonus.

Eine günstige Relation zwischen spasmolytischen Wirkungen einerseits und den bei Therapeutica mit anticholinerger Wirkkomponete auftretenden unerwinschten Wirkungen auf die Herzfrequenz, die Pupillenweite, die Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der anti-muscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von spasmolytisch wirkenden Antimuscarinica geeignet sind.

"In vitro" Organpräparationen

Dissoziationskonstanten ($K_B$-Werte) wurden "in vitro" am Ileum und spontan schlagenden Vorhof des Meerschweinchens ermittelt. Das Ileum wurde entnommen und im Organbad in Krebs-Henseleit-Lösung inkubiert. Kontraktionen wurden der art durch steigende Konzentrationen von Methacholin (M) hervorgerufen, daß volle Konzentrations-Wirkungskurven aufgenommen werden konnten. Danach wurde M ausgewaschen, die zu untersuchende Substanz beigefügt und 30 Minuten lang in Kontakt gelassen, und wiederum eine Konzentrations-Wirkungskurve mit M aufgenommen.

Aus dem Dosisverhältnis (DR), das ist das Ausmaß der Verschiebung der Konzentrations-Wirkungskurve, wurde die Dissoziationskonstante nach Arunlakshana und Schild (Brit. J. Pharmacol. 14, 48, 1959) berechnet.

Am isolierten, spontan schlagenden rechten Vorhof reduzierte M konzentrationsabhängig die Herzfrequenz. Durch Zugabe eines Antimuscarinicums wurde dieser Effekt wieder aufgehoben. Dissoziationskonstanten. für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von selektiv spasmolytisch wirkenden Substanzen. Die Ergebnisse sind in der Tabelle III enthalten.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf

1. Selektivität der bronchospasmolytischen Aktivität am Meerschweinchen,
2. Speichelsekretionshemmende Wirkung an der Ratte und
3. In-situ-spasmolytische Aktivität am Meerschweinchen untersucht.

1. Wirkung auf M-Rezeptoren der Bronchien, des Herzens und der Harnblase anaesthetisierter Meerschweinchen

Methode:

Meerschweinchen beiderlei Geschlechts (550-600 g Körpergewicht) wurden mit Urethan (1,4 g/kg, i.p.) anaesthetisiert. Eine Kanüle wurde zur Injektion der Wirkstoffe in die Jugularvene eingeführt. 220 I.U./kg Heparin wurden intravenös injiziert. Es wurde eine Kanüle in die Trachea eingeführt; die Tiere wurden künstlich mit sauerstoffangereicherter Luft mittels einer positiven Druckpumpe (Braun-Melsungen) bei einer Rate von 80 Schlägen pro Minute beatmet. Ein Ast der Trachealkanüle wurde mit einem Wassermanometer von 10 cm Höhe verbunden. Das Beatmungsvolumen wurde so eingestellt, daß der maximale intratracheale Druck während der Beatmung gerade den Druck einer 10 cm-Wassersäule erreichte.

Sieht man von einigen Modifikationen ab, wurde die Wirkung der Wirkstoffe auf den Bronchialtonus nach der von Konzett und Rössler (1940) beschriebenen Methode gemessen. Das durch Bronchokonstriktion erzeugte Volumen des Beatmungsgasgemisches (Overflow), das das Wassermanometer durchströmte, wurde mittels eines Röhren-Pneumotachometers (FLEISCH, Modell 1000), das mit einem SP 2040D Differentialdruck-Transducer (HSE) verbunden war, gemessen. Die Werte wurden mit einem IFD Aufnahmegerät registriert. Vor dem Versuch wurde die Trachea für eine kurze Zeit abgeklemmt, um den maximal möglichen Grad der Bronchokonstriktion zur Kalibrierung zu erzeugen. Eine Kanüle wurde in die linke große Halsschlagader eingeführt; der arterielle Blutdruck wurde mit Hilfe eines Drucktransducers (Bell and Howell, 4-327 I) in Verbindung mit einem IFD-Aufzeichnungsgerät gemessen. Die Herzfrequenz wurde mit einem Frequenzmesser, der durch arterielle Pulswellen ausgelöst wird, gemessen.

Es wurde ein kleiner medianer Bauchschnitt gesetzt und die Harnblase an einen Kraft-Transducer unter einer Ruhespannung von 1 Gramm angebunden.

Die zu testenden Wirksubstanzen wurden über die Jugularvene injiziert, 5 Minuten später wurde die Spannungszunahme der Blase (in Gramm), der Bronchialwiderstand (in %) und die Abnahme der Herzfrequenz (Schläge pro Minute) nach Gabe von Acetylcholin (50 µg/kg i.v. und i.a.) gemessen. Es wurden dosis-abhängige Kurven durch Wiedergabe der prozentualen Hemmung der Bronchokonstriktion, Bradycardie und der Spannungszunahme der Blase gegen den Logarithmus der Dosis (mol/kg) der zu untersuchenden Wirkstoffe aufgezeichnet. Die Ergebnisse werden angegeben als Mittelwerte (n = 4-6). Ergebnisse siehe Tabelle I.

2. Speichselsekretionshemmende Wirkung an der Ratte

Nach Lavy und Mulder (Arch. int. Pharmacodyn. 178 , 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. In-situ-spasmolytische Wirkung bei Meerschweinchen

Männliche Meerschweinchen (500 bis 600 g Körpergewicht) wurden mit Urethan (1,2 g/kg, i.p.) anaesthetisiert; es wurden Kanülen in die Trachea, Jugularvene und die linke Halsschlagader eingeführt. Die Tiere wurden künstlich mit sauerstoffangereicherter Luft mittels einer positiven Druckpumpe bei einer Schlagfrequenz von 80 pro Minute beatmet. Es wurde ein 3 bis 4 cm langer Bauchschnitt vorgenommen und etwa 15 cm einer beweglichen Schlinge des Krummdarms (Ileum) wurde unter Erhalt der Blutzirkulation distal abgebunden. Der proximale Teil wurde mit einer Krebs-Ringer-Lösung gefüllt und ein Druckmesser mit einem Millar-Mikrospitzenkatheter (PC-450, 5F) wurde in den Darm eingeführt. Eine Glasröhre wurde vertikal im Abdomen plaziert und an der umgebenden Abdominalwand befestigt in der Art, daß bei Befüllung der Glasröhre mit Krebs-Ringer-Lösung das Tier als sein eigenes Organbad diente.

Die Glasröhre wurde mit Krebs-Ringer-Lösung soweit gefüllt, daß der gesamte Unterbauch eingetaucht war. Die zu prüfenden Wirkstoffe wurden über die Jugularvene injiziert; 5 Minuten später wurden mittels Methacholin (20 µg/kg i.a.) Kontraktionen erzeugt. Durch Aufzeichnen der prozentualen Unterdrückung der durch Methacholin erzeugten Kontraktionen gegen den Logarithmus der Dosismenge (mol/kg) der zu testenden Wirksubstahz wurden Dosis-Wirkungskurven erhalten.

Die Ergebnisse wurden als Mittelwerte (n = 4 bis 8) angegeben (siehe Tabelle II).

Nach den vorstehenden Angaben wurden beispielsweise die folgenden Verbindungen untersucht:

A = L-5,11-Dihydro-11-[[6-methyl-2,6-diazabicyclo[3.3.0]oct-2-yl]carbonyl]-6H-pyrido[2,3-b][1,4]-benzodiazepin-6-on

B = D-6,11-Dihydro-11-[[6-methyl-2,6-diazabicyclo[3.3.0]oct-2-yl]carbonyl]-5H-pyrido[2,3-b][1,5]-benzodiazepin-5-on

C = 5,11-Dihydro-11-[[7-methyl-3,7-diazabicyclo[3.3.0]oct-3-yl]carbonyl]-6H-pyrido[2,3-b][1,4]-benzodiazepin-6-on

D = 4,9-Dihydro-3-methyl-4-[[7-methyl-3,7-diazabicyclo[3.3.0]oct-3-yl]carbonyl]-10H-thieno[3,4-b][1,5]-benzodiazepin-10-on

und als Vergleichssubstanzen

X = 11-[[2-[(Diethylamino)methyl]-1-piperidinyl]acetyl]-5,11-dihydro-6H-pyrido[2,3-b][1,4]benzodiazepin-6-on (siehe US-Patent 4 550 107)

Y = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380)

und

Z = Atropin.

Tabelle I:

| Selektivität der bronchospasmolytischen Aktivität am Meerschweinchen: | | | | |
|---|---|---|---|---|
| Acetylcholin - Antagonismus | | | | |
| Substanz | Bronchien -log $ED_{50}$ (mol x $kg^{-1}$) i.v. | Harnblase -log $ED_{50}$ (mol x $kg^{-1}$) i.v. | Herz -log $ED_{50}$ (mol x $kg^{-1}$) i.v. | Verhältnis Beeinflussung von Bradycardie zu Bronchokonstriktion |
| A | 7,31 | 6,31 | 5,98 | 21 |
| B | 7,35 | 6,09 | 5,71 | 44 |
| C | 6,20 | 5,52 | 5,32 | 8 |
| D | 7,05 | 5,80 | 6,13 | 8 |
| X | 5,58 | 4,93 | 5,84 | 0,5 |
| Y | 6,57 | 5,84 | 5,90 | 5 |
| Z | 8,09 | 7,28 | 7,57 | 3 |

Tabelle II:

Selektivität der in-situ-spasmolytischen Aktivität in Relation zur speichelsekretionshemmenden Wirkung.

| Substanz | In-situ-Spasmolyse Meerschweinchen-Ileum -log $ED_{50}$ (mol x $kg^{-1}$) i.v. | Speichelsekretionshemmung Ratte -log $ED_{50}$ (mol x $kg^{-1}$) i.v. | Verhältnis Speichelsekretionshemmung zur spasmolytischen Aktivität |
|---|---|---|---|
| A | 6,93 | 6,70 | 2 |
| C | 6,15 | 5,53 | 4 |
| Y | 6,08 | 6,42 | 0,5 |
| Z | 7,28 | 7,60 | 0,5 |

Tabelle III:

| Dissoziationskonstanten ($K_B$-Werte) am Ileum und spontan schlagenden Vorhof des Meerschweinchens: | | | |
|---|---|---|---|
| | $K_B$ [mol/l] | | Selektivität $K_B$ Herz zu $K_B$ Ileum |
| Substanz | Herz | Ileum | |
| A | $2{,}09.10^{-7}$ | $3{,}02.10^{-8}$ | 6,9 |
| B | $1{,}45.10^{-7}$ | $6{,}03.10^{-8}$ | 2,4 |
| X | $1{,}05.10^{-7}$ | $6{,}17.10^{-7}$ | 0,17 |
| Y | $1{,}23.10^{-7}$ | $1{,}94.10^{-7}$ | 0,63 |
| Z | $1{,}41.10^{-9}$ | $8{,}13.10^{-10}$ | 1,7 |

Diskussion der Ergebnisse:

Die Substanzen der allgemeinen Formel I hemmen in niedrigen Dosierungen die Effekte von exogen zugeführtem Acetylcholin oder Methacholin auf die glatte Muskulatur von Bronchien, Blase oder Dünndarm, ohne daß die agonistische Wirkung auf die Herzfrequenz beeinflußt wird (Tabelle I und III). Zum Beispiel zeigen die Substanzen A und B eine sehr ausgeprägte Glattmuskelselektivität; 21- und 44-fach niedrigere Dosierungen sind notwendig, um die durch Acetylcholin ausgelöste Bronchokonstriktion zu hemmen im Vergleich zu der Acetylcholin-induzierten Bradykardie (Tabelle I). Die Substanzen der allgemeinen Formel I zeigen nicht nur Selektivität für die glatte Muskulatur im Vergleich zu Effekten, die durch kardiale Muskarinrezeptoren ausgelöst werden, sondern es sind auch höhere Dosierungen erforderlich, um die Pilocarpininduzierte Speichelsekretion zu hemmen (Tabelle II).

Die beobachtete in-vivo-Selektivität der Substanzen für die glatte Muskulatur stimmt überein mit den invitro-Untersuchungen. Die Substanzen besitzen eine höhere Affinität zu Muskarinrezeptoren des Ileums im Vergleich zu kardialen Muskarinrezeptoren (Tabelle III).

Aus den Daten geht hervor, daß die Substanzen der allgemeinen Formel I die Effekte von Muskarinagonisten auf die glatte Muskulatur, z. B. Bronchien, Blase und Ileum hemmen, in Dosierungen, die keinen Einfluß auf die Herzfrequenz oder die Speichelsekretion haben. Die Vergleichssubstanzen Y (Pirenzepin) und Z (Atropin) zeigen keine Selektivität und beeinflussen die oben genannten Effekte im gleichen Dosisbereich. Die Vergleichssubstanz X zeigt eine höhere Wirksamkeit auf kardiale Muskarinrezeptoren.

Alle Substanzen der allgemeinen Formel I zeichnen sich durch eine ausgesprochene Hydrolyse-Stabilität aus. Dadurch wird es möglich, lagerbeständige Lösungen zur parenteralen Applikation herzustellen.

Die nachfolgenden Beispiele sollen die Erfindung näher erläutern:

"Fp." bedeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor. Prozente sind, sofern nichts anderes ausdrücklich erwähnt, immer Gewichtsprozente.

## Beispiel 1

### 5,11-Dihydro-11-[[7-methyl-3,7-diazabicyclo[3.3.0]-oct-3-yl]carbonyl]-6H-pyrido[2,3-b][1,4]benzodiazepin-6-on

Die Mischung aus 4,9 g (18 mMol) 11-(Chlorcarbonyl)-5,11-dihydro-6H-pyrido[2,3-b][1,4]benzodiazepin-6-on und 2,3 g 3-Methyl-3,7-diazabicyclo[3.3.0]octan in 75 ml trockenem Dimethylformamid wurde 20 Stunden lang bei Raumtemperatur gerührt. Das Reaktionsgemisch wurde im Vakuum eingeengt, der Rückstand zwischen 1N Salzsäure und Methylenchlorid verteilt. Die organische Phase wurde noch zweimal mit 1N Salzsäure und einmal mit Wasser gewaschen. Die vereinigten wässerigen Phasen wurden mit Kaliumcarbonat basisch gestellt und mit Methylenchlorid (3x150 ml) extrahiert. Die vereinigten Methylenchloridphasen wurden über Magnesiumsulfat getrocknet, eingeengt und das Rohmaterial aus Acetonitril umkristallisiert. Man erhielt 2,9 g (41 % der Theorie) an farblosen Kristallen vom Fp. 157-159 °C.

| $C_{20}H_{21}N_5O_2$ (363,43) | | | |
|---|---|---|---|
| Ber.:<br>Gef.: | C 66,10<br>65,75 | H 5,82<br>5,68 | N 19,27<br>19,17 |

## Beispiel 2

### 9-Chlor-5,11-dihydro-11-[[7-methyl-3,7-diazabicyclo[3.3.0]oct-3-yl]carbonyl]-6H-pyrido[2,3-b][1,4]-

benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 3-Methyl-3,7-diazabicyclo[3.3.0]octan in einer Ausbeute von 67 % der Theorie. Farblose Kristalle vom Fp. 216-217 °C (Acetonitril).

| $C_{20}H_{20}ClN_5O_2$ (397,87) | | | | |
|---|---|---|---|---|
| Ber.: | C 60,38 | H 5,07 | N 17,50 | Cl 8,91 |
| Gef.: | 59,73 | 5,02 | 17,28 | 8,90 |

Beispiel 3

6,11-Dihydro-11-[[7-methyl-3,7-diazabicyclo[3.3.0]oct-3-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 3-Methyl-3,7-diazabicyclo[3.3.0]octan in einer Ausbeute von 7 % der Theorie. Farblose Kristalle vom Fp. 115-120 °C (Essigester/Diisopropylether).

Beispiel 4

4,9-Dihydro-3-methyl-4-[[7-methyl-3,7-diazabicyclo[3.3.0]oct-3-yl]carbonyl]-10H-thieno[3,4-b][1,5]-benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 3-Methyl-3,7-diazabicyclo[3.3.0]octan in einer Ausbeute von 13 % der Theorie. Farblose Kristalle vom Fp. 220-222 °C (Ethanol).

Beispiel 5

3-Chlor-1-methyl-4-[[7-methyl-3,7-diazabicyclo[3.3.0]oct-3-yl]carbonyl]-1.4.9.10-tetrahydropyrrolo[3,2-b][1,5]-benzodiazepin-10-on

Hergestellt analog Beispiel 1 aus 3-Chlor-4-(chlorcarbonyl)-4,9-dihydro-1-methyl-1.4.9.10-tetrahydropyrrolo[3,2-b][1,5]benzodiazepin-10-on und 3-Methyl-3,7-diazabicyclo[3.3.0]octan in einer Ausbeute von 24 % der Theorie. Farblose Kristalle vom Fp. >280 °C (Ethanol/Wasser).

Beispiel 6

L-5,11-Dihydro-11-[[6-methyl-2,6-diazabicyclo[3.3.0]oct-2-yl]carbonyl]-6H-pyrido[2,3-b][1,4]benzodiazepin-6-on

a.) L-2-Benzyl-6-ethoxycarbonyl-2,6-diazabicyclo[3.3.0]octan

Zu der Mischung aus 5,7 g (28,2 mMol) L-2-Benzyl-2,6-diazabicyclo[3.3.0]octan, 3,1 g (31,0 mMol) Triethylamin in 0,5 l Ether wurde Chlorameisensäureethylester (3,7 g, 33,8 mMol) tropfenweise zugegeben und über Nacht gerührt. Das entstandene Festmaterial wurde abfiltriert und das Filtrat eingeengt. Das

Rohprodukt (6,0 g, 81 % der Theorie) wurde ohne weitere Reinigung in der folgenden Stufe verwendet. Rf = 0,7 (Merck, DC-Fertigplatten, Kieselgel 60 $F_{254}$; Fließmittel: Dichlormethan/Methanol/konz. Ammoniak 90/10/1, v/v/v).

b.) L-6-Methyl-2,6-diazabicyclo[3.3.0]octan

Die Mischung aus 6,0 g (22,8 mMol) L-2-Benzyl-6-ethoxycarbonyl-2,6-diazabicyclo[3.3.0]octan und 0,87 g (22,8 mMol) Lithiumaluminiumhydrid in 200 ml Ether wurde 5 Stunden unter Rückfluß erhitzt. Unter Eiskühlung wurden anschließend 5 ml 30%-ige wässrige Natronlauge zugetropft und vom weißen Niederschlag abdekantiert. Die Etherlösung wurde getrocknet und eingeengt, das zurückbleibende ölige L-2-Benzyl-6-methyl-2,6-diazabicyclo[3.3.0]octan (4,8 g, 97 % der Theorie) war nach dünnschichtchromatographischer Analyse [Rf = 0,35 (Merck, DC-Fertigplatten, Kieselgel 60 $F_{254}$; Fließmittel: Dichlormethan/methanol/konz. Ammoniak 90/10/1, v/v/v)] einheitlich und wurde im Autoklaven unter Zugabe von 0,5 g Palladium auf Kohle (10 %) und in Äthanol 5 Stunden bei 60 °C hydriert. Das Reaktionsgemisch wurde unter vermindertem Druck eingeengt und als Rohprodukt (3,3 g) für die weiteren Umsetzungen benutzt.

c.) L-5,11-Dihydro-11-[[6-methyl-2,6-diazabicyclo[3.3.0]oct-2-yl]carbonyl]-6H-pyrido[2,3-b][1,4]-benzodiazepin-6-on

Die Mischung aus 0,95 g (3,5 mMol) 11-(Chlorcarbonyl)-5,11-dihydro-6H-pyrido[2,3-b][1,4]-benzodiazepin-6-on und 0,40 g (3,2 mMol) L-2-Methyl-2,6-diazabicyclo[3.3.0]octan wurde 3 Stunden lang in 50 ml Acetonitril gerührt, anschließend unter vermindertem Druck eingeengt. Der Rückstand wurde zwischen Wasser und Essigester, in dem zuvor 0,4 g (3,5 mMol) Maleinsäure aufgelöst wurde, verteilt. Die wässrige Phase wurde noch weitere zweimal mit Essigester nachextrahiert und durch Zugabe von 0,5 g Kaliumcarbonat alkalisch gestellt. Die wässrige Phase wurde mit Dichlormethan erschöpfend extrahiert und die vereinigten organischen Extrakte getrocknet und eingeengt. Der Rückstand wurde säulenchromatographisch (Silicagel 63-200 µm; Laufmittel: Dichlormethan/Methanol/konz. Ammoniak 1200/50/5, v/v/v) gereinigt. Man erhielt 0,44 g (38 % der Theorie) Kristalle vom Fp. 220-222 °C (Acetonitril).

Beispiel 7

L-5,11-Dihydro-8-methyl-11-[[6-methyl-2,6-diazabicyclo[3.3.0]oct-2-yl]carbonyl]-6H-pyrido[2,3-b][1,4]-benzodiazepin-6-on

Hergestellt analog Beispiel 6c aus 2,0 g (7,0 mMol) 11-(Chlorcarbonyl)-5,11-dihydro-8-methyl-6H-pyrido[2,3-b][1,4] benzodiazepin-6-on, 0,8 g (6,3 mMol) L-2-Methyl-2,6-diazabicyclo[3.3.0]octan und 100 ml Acetonitril in einer Ausbeute von 25 %. Farblose Kristalle vom Fp. 160-165 °C.

Beispiel 8

L-6,11-Dihydro-11-[[6-methyl-2,6-diazabicyclo[3.3.0]oct-2-yl]carbonyl]-5H-pyrido[2,3-b][1,5]benzodiazepin-5-on

Hergestellt analog Beispiel 6c aus 1,9 g (7,0 mMol) 11-(Chlorcarbonyl)-6,11-dihydro-5H-pyrido[2,3-b]-[1,5]benzodiazepin-5-on, 0,8 g (6,3 mMol) L-2-Methyl-2,6-diazabicyclo[3.3.0]octan und 100 ml Acetonitril in einer Ausbeute von 54 % der Theorie.
$[\alpha]_D^{20} = +314^\circ$.

Beispiel 9

D-5,11-Dihydro-11-[[6-methyl-2,6-diazabicyclo[3.3.0]oct-2-yl]carbonyl]-6H-pyrido[2,3-b][1,4]benzodiazepin-6-on

Hergestellt analog Beispiel 6c aus 0,95 g (3,5 mMol) 11-(Chlorcarbonyl)-5,11-dihydro-6H-pyrido[2,3-b]-[1,4]benzodiazepin-6-on, 0,40 g (3,2 mMol) D-2-Methyl-2,6-diazabicyclo[3.3.0]octan in 50 ml Acetonitril. Farblose Kristalle vom Fp. 160-165 °C in einer Ausbeute von 38 % der Theorie.

Beispiel 10

D-5,11-Dihydro-8-ethyl-11-[[6-methyl-2,6-diazabicyclo[3.3.0]oct-2-yl]carbonyl]-6H-pyrido[2,3-b][1,4]-benzodiazepin-6-on

Hergestellt analog Beispiel 6c aus 1,6 g (5,3 mMol) 8-Ethyl-11-(chlorcarbonyl)-5,11-dihydro-6H-pyrido-[2,3-b][1,4]benzodiazepin-6-on, 0,67 g (5,3 mMol) D-2-Methyl-2,6-diazabicyclo[3.3.0]octan in 67%-iger Ausbeute.
Farblose Kristalle vom Fp. 162-165 °C.

Beispiel 11

D-6,11-Dihydro-11-[[6-methyl-2,6-diazabicyclo[3.3.0]oct-2-yl]carbonyl]-5H-pyrido[2,3-b][1,5]benzodiazepin-5-on

Hergestellt analog Beispiel 6c aus 0,95 g (3,5 mMol) 11-(Chlorcarbonyl)-6,11-dihydro-5H-pyrido[2,3-b]-[1,5]benzodiazepin-5-on, 0,40 g (3,2 mMol) D-2-Methyl-2,6-diazabicyclo[3.3.0]octan in einer Ausbeute von 42 %. Farblose Kristalle vom Fp. 172-174 °C, $[\alpha]_D^{20}$ = + 284 °.

Beispiel 12

L-5,11-Dihydro-11-[[6-isopropyl-2,6-diazabicyclo[3.3.0]oct-2-yl]carbonyl-6H-pyrido[2,3-b][1,4]benzodiazepin-6-on

a.) L-2-Isopropyl-2,6-diazabicyclo[3.3.0]octan

Die Mischung aus 10,0 g (34 mMol) L-2,6-Dibenzyl-2,6-diazabicyclo[3.3.0]octan, 10,0 g Aceton (172 mMol) und Ethanol (1,0 Liter) wurde unter Zusatz von 1 g Palladium auf Kohle (10 %) 20 Stunden lang bei 60 °C hydriert. Der Katalysator wurde abfiltriert und das Reaktionsgemisch eingeengt. Der Rückstand wurde säulenchromatographisch (Silicagel 60-200 µm; Laufmittel: Dichlormethan/Methanol/Konz. Ammoniak 100/10/1, v/v/v) gereinigt. Es wurden 1,2 g (26 % der Theorie) L-2-Isopropyl-2,6-diazabicyclo[3.3.0]octan und 1,5 g (45 % der Theorie) 2,6-Diazabicyclo[3.3.0]octan eluiert.
L-2-Isopropyl-2,6-diazabicyclo[3.3.0]octan.
Rf = 0,2 (Merck, DC-Fertigplatten, Kieselgel 60 $F_{254}$; Fließmittel: Dichlormethan/Methanol/konz. Ammoniak 150/50/5, v/v/v).

b.) L-5,11-Dihydro-11-[[6-isopropyl-2,6-diazabicyclo[3.3.0]oct-2-yi]carbonyl]-6H-pyrido[2,3-b][1,4]-benzodiazepin-6-on

Hergestellt analog Beispiel 6c aus 2,1 g (7,8 mMol) 11-(Chlorcarbonyl)-5,11-dihydro-6H-Pyrido[2,3-b]-[1,4]benzodiazepin-6-on und 1,2 g (7,8 mMol) L-2-Isopropyl-2,6-diazabicyclo[3.3.0]octan in 50 ml trockenem Dimethylformamid in einer Ausbeute von 56 % der Theorie.
Rf = 0,2 (Merck, DC-Fertigplatten, Kieselgel 60 $F_{254}$; Fließmittel: Dichlormethan/Methanol/konz. Ammoniak

140/10/1, v/v/v).

| | | | |
|---|---|---|---|
| Ber.: | C 67,50 | H 6,44 | N 17,89 |
| Gef.: | 67,66 | 6,65 | 17,36 |

Beispiel 13

cis-5,11-Dihydro-11-[[8-methyl-2,8-diazabicyclo[4.4.0]dec-2-yl]carbonyl]-6H-pyrido[2,3-b][1,4]benzodiazepin-6-on und

trans-5,11-Dihydro-11-[[8-methyl-2,8-diazabicyclo[4.4.0]dec-2-yl]carbonyl]-6H-pyrido[2,3-b][1,4]-benzodiazepin-6-on

Die Mischung aus 1,64 g (5,0 mMol) 11-(Chlorcarbonyl)-5,11-dihydro-6H-pyrido[2,3-b][1,4]-benzodiazepin-6-on, 0,8 g (5,2 mMol) 8-Methyl-2,8-diazabicyclo[4.4.0]decan und 0,85 ml (6,0 mMol) Triethylamin in 20 ml Tetrahydrofuran wurde 3 Stunden bei Raumtemperatur gerührt. Das Reaktionsgemisch wurde im Vakuum eingeengt und der Rückstand zwischen Essigester und verdünnter wässriger Kaliumcarbonatlösung verteilt. Die organische Phase wurde abgetrennt, getrocknet und im Vakuum eingeengt. Die chromatographische Reinigung erfolgte auf Kieselgel (30-60 µm) mit dem Lösungsmittelgemisch Essigester/Methanol/konz. Ammoniak 70/30/1, v/v/v. Die beiden Hauptfraktionen wurden eingeengt, mit Acetonitril verrieben und der entstehende Niederschlag abfiltriert. Aus der zuerst eluierten Hauptfraktion erhielt man 100 mg (5 % der Theorie) an Kristallen vom Fp. 230-233 °C, die mit spektroskopischen Methoden als trans-Verbindung identifiziert wurde. Die nachfolgende Hauptfraktion enthielt 200 mg (10 % der Theorie) der kristallinen cis-Verbindung mit Fp. 160-161 °C.

Beispiel 14

5,11-Dihydro-11-[[10-methyl-4,10-diazatricyclo[5.2.1.$0^{2,6}$]dec-4-yl]carbonyl]-6H-pyrido[2,3-b][1,4]-benzodiazepin-6-on

Die Mischung aus 1,44 g (5,25 mMol) 11-(Chlorcarbonyl)-5,11-dihydro-6H-pyrido[2,3-b][1,4]-benzodiazepin-6-on, 0,8 g (5,25 mMol) 10-Methyl-4,10-diazatricyclo[5.2.1.$0^{2,6}$]decan und 0,73 ml (5,25 mMol) Triethylamin wurde 8 Stunden bei Raumtemperatur in 80 ml Acetonitril gerührt. Die Suspension wurde vom Festkörper abgesaugt und das erhaltene Filtrat eingeengt. Der Rückstand wurde aus Essigester umkristallisiert, die Kristalle in Methylenchlorid aufgenommen und mit wässriger Natriumcarbonatlösung behandelt. Dann wurde die organische Phase abgetrennt und die wässrige Phase noch zweimal mit Methylenchlorid extrahiert. Die vereinigten organischen Phasen wurden über Natriumsulfat getrocknet, filtriert und im Vakuum eingeengt. Der Rückstand wurde schließlich über eine Kieselgelsäule (Kieselgel 30-60 µm; Laufmittel: Cyclohexan/Essigester/Methanol/konz. Ammoniak 2/2/10/1, v/v/v/v) gereinigt und anschließend nochmals aus Essigester umkristallisiert. Man erhielt 0,53 g (26 % der Theorie) an weißen Kristallen vom Fp. 218-220 °C.

| $C_{22}H_{23}N_5O_2$ (389,46) | | | |
|---|---|---|---|
| Ber.: | C 67,85 | H 5,95 | N 17,98 |
| Gef.: | 67,98 | 6,10 | 18,18 |

Beispiel 15

5,10-Dihydro-5-[[10-methyl-4,10-diazatricyclo[5.2.1.$0^{2,6}$]dec-4-yl]carbonyl]-11H-dibenzo[b,e][1,4]diazepin-11-on

Hergestellt analog Beispiel 14 aus 1,40 g (5,1 mMol) 5-(Chlorcarbonyl)-5,10-dihydro-11H-dibenzo[b,e]-[1,4]diazepin-11-on, 0,78 g (5,12 mMol) 10-Methyl-4,10-diazatricyclo[5.2.1.$0^{2,6}$]decan, 0,7 ml (5,1 mMol) Triethylamin und 60 ml Acetonitril in einer Ausbeute von 30 % der Theorie. Farblose Kristalle vom Fp. 230-233° C.

| $C_{22}H_{24}N_4O_2$ (388,47) | | | |
| --- | --- | --- | --- |
| Ber.: | C 71,11 | H 6,23 | N 14,42 |
| Gef.: | 70,75 | 6,34 | 14,42 |

Beispiel 16

L-4,9-Dihydro-3-methyl-4-[[6-methyl-2,6-diazabicyclo[3.3.0]oct-2-yl]carbonyl]-10H-thieno[3,4-b][1,5]-benzodiazepin-10-on

Hergestellt ananlog Beispiel 6c aus 4-(Chlorcarbonyl)-4,9-dihydro-3-methyl-10H-thieno[3,4-b][1,5]-benzodiazepin-10-on und L-2-Methyl-2,6-diazabicyclo[3.3.0]octan in einer Ausbeute von 37 % der Theorie. Farblose Kristalle vom Fp. 95-100° C (Acetonitril)

Beispiel 17

L-3-Chlor-1-methyl-4-[[6-methyl-2,6-diazabicyclo[3.3.0]oct-2-yl]carbonyl]-1,4,9,10-tetrahydropyrrolo[3,2-b]-[1,5]benzodiazepin-10-on

Hergestellt analog Beispiel 6c aus 3-Chlor-4-(Chlorcarbonyl)-1-methyl-1,4,9,10-tetrahydropyrrolo[3,2-b]-[1,5]benzodiazepin-10-on und L-2-Methyl-2,6-diazabicyclo[3.3.0]octan in einer Ausbeute von 56 % der Theorie. Farblose Kristalle vom Fp. 200-203° C.

Beispiel 18

trans-5,11-Dihydro-11-[[7-methyl-2,7-diazabicyclo[4.4.0]dec-2-yl]carbonyl-6H-Pyrido[2,3-b][1,4]-benzodiazepin-6-on

a.) trans-2,7-Diazabicyclo[4.4.0]decan

1,5-Naphthyridin (5,0 g, 38,4 mMol) in 450 ml Amylalkohol wurde am Rückfluß erhitzt und Natrium (21,3 g, 0,92 Mol) portionsweise über einen Zeitraum von 30 Minuten zu der Reaktionsmischung gegeben. Anschließend wurden 84 ml konzentrierte Salzsäure zugegeben. Die organische Phase wurde abgetrennt, zweimal mit Wasser gewaschen und die vereinigten wässrigen Extrakte noch. zweimal mit Ether extrahiert. Die Etherphasen wurden verworfen, die wässrige Phase mit Natronlauge unter Eiskühlung basisch gestellt und das gewünschte Produkt durch mehrmalige Extraktion mit Dichlormethan ausgezogen. Die vereinigten Dichlormethanphasen wurden getrocknet und unter vermindertem Druck eingeengt. Quantitiative Ausbeute (5,8 g) von Kristallen vom Fp. 174-176° C (Essigester).

b.) trans-2-Methyl-2,7-diazabicyclo[4.4.0]decan

Eine Mischung aus trans-2,7-Diazabicyclo[4.4.0]decan (1,4 g, 10 mMol), 37%iger wässeriger Formaldehydlösung (0,6 ml, 8 mMol) und 0,5 g Palladium auf Kohle (10 %) wurde 10 Stunden bei 60 °C unter einem Druck von 3 bis 4 bar hydriert. Nach Zugabe von weiteren 0,3 ml der 37%igen wässerigen Formaldehydlösung wurde unter den oben angegebenen Bedingungen weiter hydriert, das Reaktionsgemisch anschließend eingeengt und mit Petrolether digeriert. Die Petroletherlösung wurde unter vermindertem Druck eingeengt und der ölige Rückstand (1,2 g) ohne weitere Reinigung für die weiteren Umsetzungen benutzt.

c.) trans-5,11-Dihydro-11-[[7-methyl-2,7-diazabicyclo[4.4.0]dec-2-yl]carbonyl-6H-Pyrido[2,3-b][1,4]-benzodiazepin-6-on

Die Mischung aus 0,84 g (3,1 mMol) 11-(Chlorcarbonyl)-5,11-dihydro-6H-Pyrido[2,3-b][1,4]-benzodiazepin-6-on und 0,43 g (2,8 mMol) trans-2-Methyl-2,7-diazabicyclo[4.4.0]decan wurde 24 Stunden lang in 80 ml Acetonitril gerührt und anschließend unter vermindertem Druck eingeengt. Der Rückstand wurde zwischen Wasser und Essigester, in dem zuvor 0,3 g Maleinsäure aufgelöst wurde, verteilt. Die wässrige Phase wurde noch einmal mit Essigester nachextrahiert, durch Zugabe von Kaliumcarbonat alkalisch gestellt und dann mit Dichlormethan erschöpfend extrahiert. Die vereinigten organischen Extrakte wurden getrocknet, eingeengt und säulenchromatographisch (Silicagel 63-200 µm, Laufmittel: Dichlormethan/Methanol/konz. Ammoniak 90/10/1, v/v/v/) gereinigt. Man erhielt 0,2 g der Titelverbindung (18 % der Theorie).

Beispiel 19

trans-6,11-Dihydro-11-[[6-methyl-2,6-diazabicyclo[4.4.0]-dec-2-yl]carbonyl]-5H-pyrido[2,3-b][1,5]-benzodiazepin-5-on

Hergestellt analog Beispiel 18c aus 0,84 g (3,1 mMol) 11-(Chlorcarbonyl)-6,11-dihydro-5H-pyrido[2,3-b]-[1,5]benzodiazepin-5-on, 0,43 g (2,8 mMol) trans-2-Methyl-2,7-diazabicyclo[4.4.0]decan in einer Ausbeute von 37 %.

Beispiel 20

L-5,11-Dihydro-11-[[6-methyl-2,6-diazabicyclo[3.3.0]oct-2-yl]carbonyl]-6H-pyrido[2,3-b][1,4]benzodiazepin-6-on

In eine Mischung, bestehend aus 11,2 ml einer 20 eigen Lösung von Phosgen in Toluol, 50 ml Acetonitril und 2,4 g (0,023 mol) wasserfreiem Natriumcarbonat wurden unter äußerer Kühlung mit Eis 2,6 g (0,021 mol) L-2-Methyl-2,6-diazabicyclo[3.3.0]octan zugetropft. Nach 60 Minuten wurde 4,5 g (0,021 mol) 5,11-Dihydro-6H-pyrido[2,3-b][1,4]benzodiazepin6-on zugegeben und 4 Stunden unter Rückfluß gekocht. Die heiße Mischung wurde filtriert, der Niederschlag dreimal mit je 10 ml heißem Acetonitril gewaschen und die vereinigten Filtrate im Vakuum auf ein Gesamtvolumen von 15 ml eingeengt. Die Lösung wurde im Eisbad gekühlt und der entstehende Kristallbrei abgenutscht. Umkristallisation aus Acetonitril ergab 2,1 g (27 % der Theorie) farblose Kristalle vom Fp. 220-222 °C, nach Mischschmelzpunkt und spektroskopischen Daten identisch mit dem nach Beispiel 6c hergestellten Präparat.

Entsprechend erhielt man:

5,11-Dihydro-11-[[7-methyl-3,7-diazabicyclo[3.3.0]oct-3-yl]carbonyl]-6H-pyrido[2,3-b][1,4]benzodiazepin-6-on vom Fp. 157-159 °C (Acetonitril)

5,11-Dihydro-11-[[10-methyl-4,10-diazatricyclo[5.2.1 6]dec-4-yl]carbonyl]-6H-pyrido[2,3-b][1,4]-benzodiazepin-6-on vom Fp. 218-220 °C (Essigester).

Im folgenden wird die Herstellung pharmazeutischer Anwendungsformen anhand einiger Beispiele beschrieben:

Beispiel I

Tabletten mit 5 mg D-6,11-Dihydro-11-[[6-methyl-2,6-diazabicyclo[3.3.0]oct-2-yl]carbonyl]-5H-pyrido[2,3-b]-[1,5]benzodiazepin-5-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.
Tablettengewicht: 220 mg
Stempel: 9 mm

Beispiel II

Dragées mit 5 mg D-6,11-Dihydro-11-[[6-methyl-2,6-diazabicyclo[3.3.0]oct-2-yl]carbonyl]-5H-pyrido[2,3-b]-[1,5]benzodiazepin-5-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg D-6,11-Dihydro-11-[[6-methyl-2,6-diazabicyclo[3.3.0]oct-2-yl]carbonyl]-5H-pyrido[2,3-b][1,5]benzodiazepin-5-on

| Zusammensetzung: | | |
|---|---|---|
| 1 Ampulle enthält: | | |
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120° C.

Beispiel IV

Suppositorien mit 20 mg D-6,11-Dihydro-11-[[6-methyl-2,6-diazabicyclo[3.3.0]oct-2-yl]carbonyl]-5H-pyrido-[2,3-b][1,5]benzodiazepin-5-on

| Zusammensetzung: | |
|---|---|
| 1 Zäpfchen enthält: | |
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht 1,7 g

Beispiel V

Tropfen mit D-6,11-Dihydro-11-[[6-methyl-2,6-diazabicyclo[3.3.0]oct-2-yl]carbonyl]-5H-pyrido[2,3-b][1,5]-benzodiazepin-5-on

| Zusammensetzung: | | |
|---|---|---|
| 100 ml Tropflösung enthalten: | | |
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Ansprüche

1.) Kondensierte Diazepinone der allgemeinen Formel I

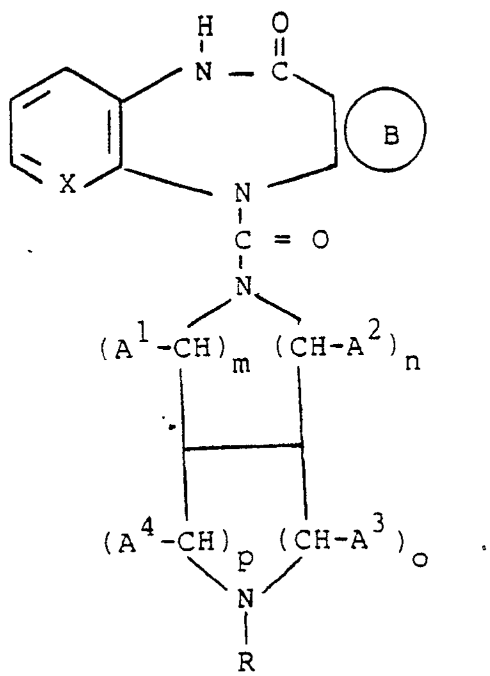

(I)

in der

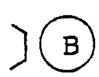

einen der zweiwertigen Reste

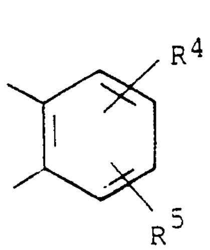 , 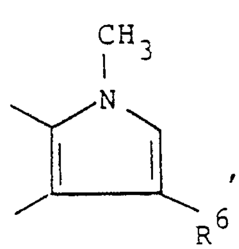 , 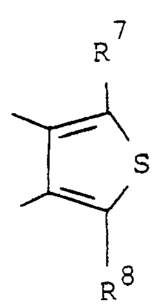 , 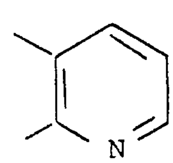

(S) (T) (U) (V)

darstellt und
X, R, $R^4$, $R^5$, $R^6$, $R^7$, $R^8$, m, n, o, p, $A^1$, $A^2$, $A^3$ und $A^4$ die folgenden Bedeutungen besitzen:
X ist eine =CH-Gruppe oder ein Stickstoffatom,
R ist ein geradkettiger oder verzweigter Alkylrest mit 1 bis 4 Kohlenstoffatomen, der gegebenenfalls noch

durch ein durch Chlor, Brom, Fluor, Methyl oder Methoxy mono- oder disubstituiertes Phenyl substituiert sein kann,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 0, 1, 2 oder 3 mit folgenden Einschränkungen:

die Summe aus m + n und die Summe aus o + p bedeutet jeweils die Zahlen 1, 2 oder 3,

die Summe aus n + o und die Summe aus m + p bedeutet jeweils die Zahlen 1, 2, 3, 4 oder 5,

wobei jedoch die Summe aus m + n + o + p immer größer als 2 sein muß,

$A^1$, $A^2$, $A^3$, $A^4$ bedeuten Wasserstoffatome; für den Fall, daß m, n, o und p jeweils die Zahl 1 bedeuten, können entweder $A^1$ und $A^2$ zusammen oder $A^3$ und $A^4$ zusammen auch eine Ethylenbrücke darstellen,

ihre Isomeren und ihre physiologisch verträglichen Salze mit anorganischen oder organischen Säuren.

2.) Kondensierte Diazepinone der allgemeinen Formel I gemäß Anspruch 1, in der entweder

X ein Stickstoffatom und

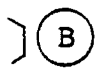

den Rest (S) oder

X die = CH-Gruppe und

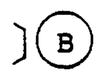

den Rest (V) bedeuten,

R die Methylgruppe,

$R^4$ und $R^5$, die gleich oder voneinander verschieden sein können, jeweils ein Wasserstoff-, Fluor- oder Chloratom, die Methyl- oder Ethylgruppe darstellen und

m = 0, n = 2, o = 0, p = 2 oder

m, n, o und p jeweils gleich 1 sind,

ihre Isomeren und ihre physiologisch verträglichen Salze mit anorganischen oder organischen Säuren.

3.) Als kondensierte Diazepinone der allgemeinen Formel I gemäß Anspruch 1 die Verbindungen

L-5,11-Dihydro-11-[[6-methyl-2,6-diazabicyclo[3.3.0]oct-2-yl]carbonyl]-6H-pyrido[2,3-b][1,4]benzodiazepin-6-on

D-6,11-Dihydro-11-[[6-methyl-2,6-diazabicyclo[3.3.0]oct-2-yl]carbonyl]-5H-pyrido[2,3-b][1,5]benzodiazepin-5-on

5,11-Dihydro-11-[[7-methyl-3,7-diazabicyclo[3.3.0]oct-3-yl]carbonyl]-6H-pyrido[2,3-b][1,4]benzodiazepin-6-on

ihre Isomeren und ihre physiologisch verträglichen Salze mit anorganischen oder organischen Säuren.

4.) Arzneimittel enthaltend eine oder mehrere Verbindungen gemäß den Ansprüchen 1 bis 3 neben den üblichen Träger-und/oder Hilfsstoffen.

5.) Verwendung von Verbindungen gemäß Anspruch 1 bis 3 zur Herstellung von Arzneimitteln zur Therapie cholinerg bedingter Spasmen und Motilitätsstörungen im Magen-Darm-Trakt und im Bereich der abführenden Gallengänge, zur Therapie der Cystitis und von Spasmen bei Urelithiasis, zur Therapie der relativen Inkontinenz, zur Therapie des Asthma bronchiale und der Bronchitis und zur Therapie ischämischer Herzerkrankungen.

6.) Verfahren zur Herstellung von kondensierten Diazepinonen der allgemeinen Formel I

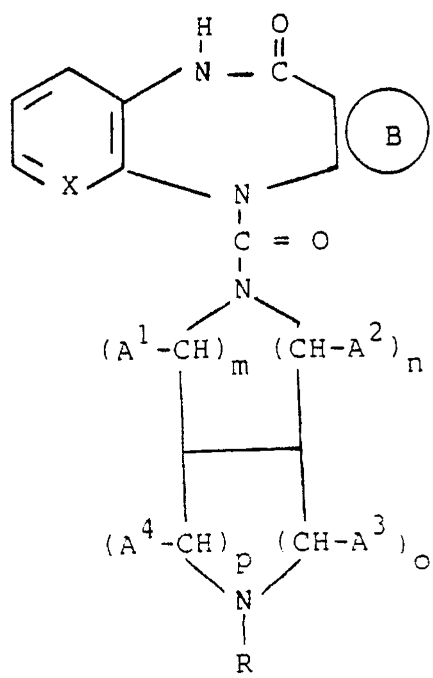

(I)

in der

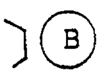

einen der zweiwertigen Reste

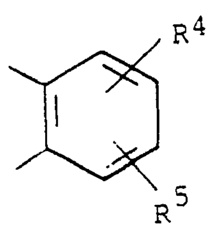

,

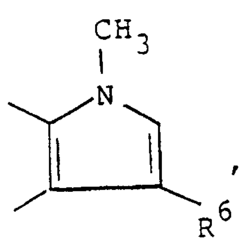

,

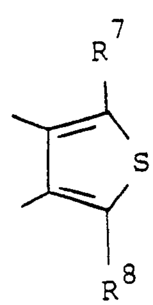

,

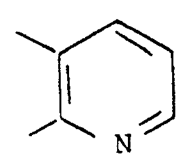

(S) (T) (U) (V)

darstellt und
X, R, $R^4$, $R^5$, $R^6$, $R^7$, m, n, o, p, $A^1$, $A^2$, $A^3$ und $A^4$
die folgenden Bedeutungen besitzen:
X ist eine =CH-Gruppe oder ein Stickstoffatom,
R ist ein geradkettiger oder verzweigter Alkylrest mit 1 bis 4 Kohlenstoffatomen, der gegebenenfalls noch durch ein durch Chlor, Brom, Fluor, Methyl oder Methoxy mono- oder disubstituiertes Phenyl substituiert sein kann,
$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,
$R^6$ ist ein Wasserstoff- oder Chloratom oder eine Methylgruppe,
$R^7$ und die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,
m, n, o und p bedeuten jeweils die Zahlen 0, 1, 2 oder 3 mit folgenden Einschränkungen:
die Summe aus m+n und die Summe aus o+p bedeutet jeweils die Zahlen 1, 2 oder 3,
die Summe aus n+o und die Summe aus m+p bedeutet jeweils die Zahlen 1, 2, 3, 4 oder 5,
wobei jedoch die Summe aus m+n+o+p immer größer als 2 sein muß,

$A^1$, $A^2$, $A^3$, $A^4$ bedeuten Wasserstoffatome; für den Fall, daß m, n, o und p jeweils die Zahl 1 bedeuten, können entweder $A^1$ und $A^2$ zusammen oder $A^3$ und $A^4$ zusammen auch eine Ethylenbrücke darstellen, ihrer Isomeren und ihrer Salze mit anorganischen oder organischen Säuren, dadurch gekennzeichnet, daß a.) zur Herstellung basisch substituierter kondensierter Diazepinone der allgemeinen Formel Ia

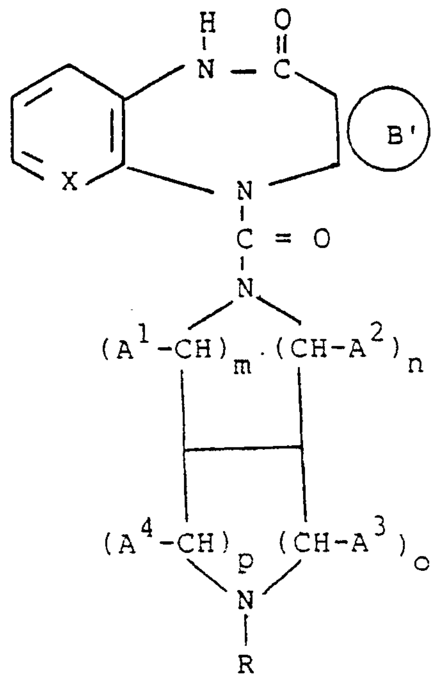

(Ia)

in der
X, R, $R^4$, $R^5$, $R^6$, $R^7$, $R^8$, m, n, o, p, $A^1$, $A^2$, $A^3$, $A^4$ die oben angegebenen Bedeutungen haben und

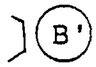

einen der zweiwertigen Reste (S), (U), (V) oder (T′)

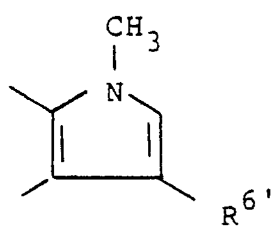

(T')

darstellt, worin $R^4$, $R^5$, $R^7$ und wie oben definiert sind und
$R^{6'}$ ein Chloratom oder eine Methylgruppe bedeutet, ein Kohlensäurederivat der allgemeinen Formel II

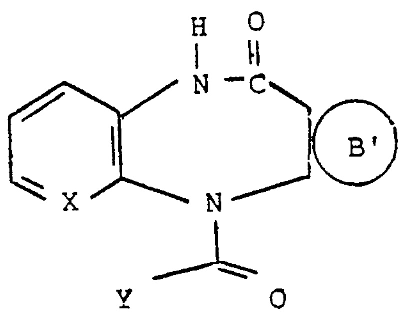

(II)

in der

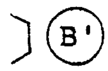

und X wie oben definiert sind und

Y ein Halogenatom oder den Rest $-OR^{11}$ bedeutet, in dem $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit einer Verbindung der allgemeinen Formel III

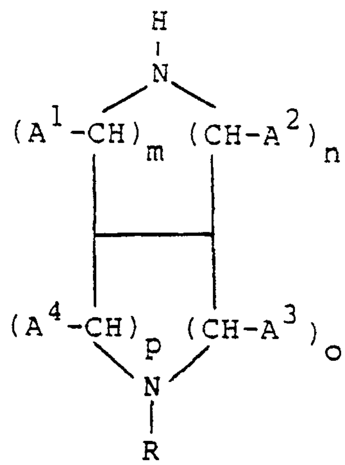

(III)

oder mit ihrer Metallvervindung der allgemeinen Formel IIIa

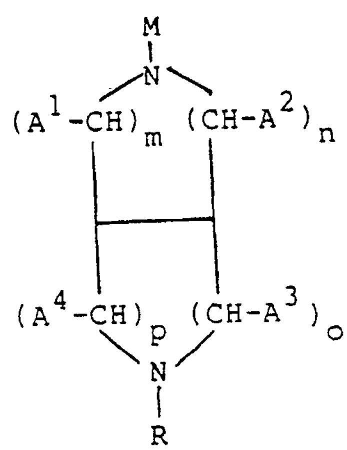

(IIIa)

in welchen R, m, n, o, p, $A^1$, $A^2$, $A^3$, $A^4$ wie oben definiert sind und M ein Alkalimetallatom oder 1 Äquivalent eines Erdalkalimetallatoms bedeutet, gegebenennfalls in Anwesenheit eines Lösungsmittels, bei Temperaturen zwischen -10 °C und dem Siedepunkt des Reaktionsgemisches, gegebenenfalls in Gegenwart einer Base oder eines Überschußes der Verbindung der allgemeinen Formel III, umgesetzt wird, oder

b.) zur Herstellung basisch substituierter kondensierter Diazepinone der vorgenannten allgemeinen Formel Ia

ein Tricyclus der allgemeinen Formel IV

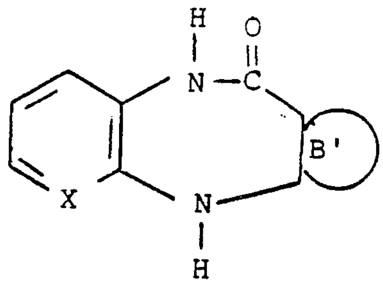

(IV)

in der
X und

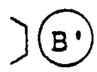

wie oben definiert sind, mit einem Chlorkohlensäurederivat der allgemeinen Formel V

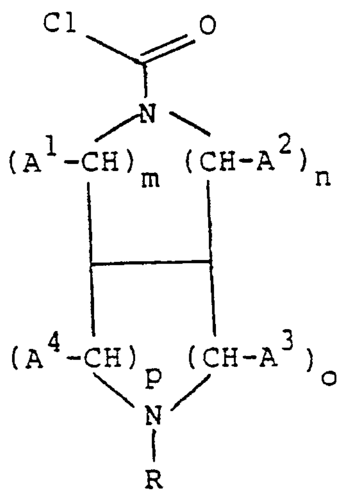

(V)

in der
R, m, n, o und p, $A^1$, $A^2$, $A^3$ und $A^4$ die oben erwähnten Bedeutungen haben,
in inerten Lösungsmitteln, vorzugsweise in Gegenwart von Basen, bei Temperaturen zwischen 30 und 100° C umgesetzt werden, oder
c.) zur Herstellung von unter die allgemeine Formel I fallenden Pyrrolo-kondensierten Diazepinonen der allgemeinen Formel Ib

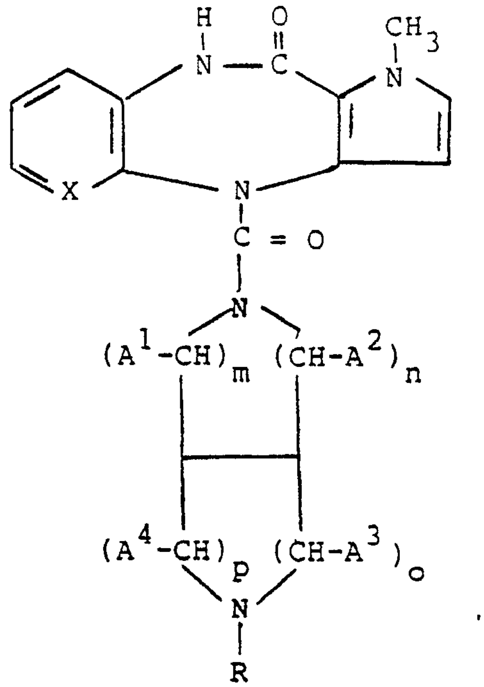

(Ib)

in der
X, R, m, n, o, p, $A^1$, $A^2$, $A^3$ und $A^4$ die oben erwähnten Bedeutungen besitzen, Verbindungen der allgemeinen Formel Ia, in der

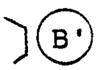

den zweiwertigen Rest (T′)

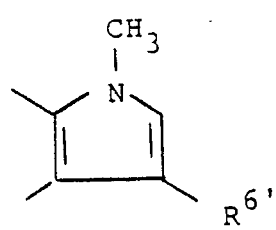

(T')

bedeutet, worin $R^{6'}$ ein Chloratom darstellt, einer Hydrogenolyse unterworfen werden, und, gegebenenfalls anschließend, so erhaltene Verbindungen der allgemeinen Formel I in ihre Isomeren aufgetrennt und/oder mit anorganischen oder organischen Säuren in ihre Salze übergeführt werden.

7.) Verfahren gemäß Anspruch 6c, dadurch gekennzeichnet, daß die Hydrogenolyse in Gegenwart von Katalysatoren von Metallen der VIII. Nebengruppe des Periodensystem, bei Wasserstoffdrucken von 1 bis 300 bar und Temperaturen von 0° bis 130° C in Gegenwart von Lösungsmitteln durchgeführt wird.

8.) Verfahren gemäß Anspruch 6c, dadurch gekennzeichnet, daß die Hydrogenolyse

a.) mit Ameisensäure und einem Palladium-auf-Kohle-Katalysator bei Temperaturen zwischen 70 und 110° C in Gegenwart eines Lösungsmittels oder

b.) mit Triethylammoniumformiat in Gegenwart überschüssigen Triethylamins und von Palladium auf Tierkohle oder

c.) mit Palladiumacetat und Triarylphosphinen bei Temperaturen zwischen 40 und 110° C erfolgt.

Patentansprüche für folgende Vertragsstaaten: ES, GR

1.) Verfahren zur Herstellung von kondensierten Diazepinonen der allgemeinen Formel I

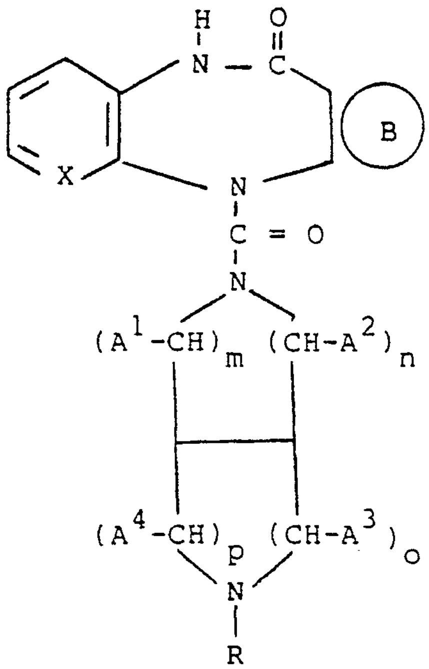

(I)

in der

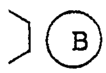

einen der zweiwertigen Reste

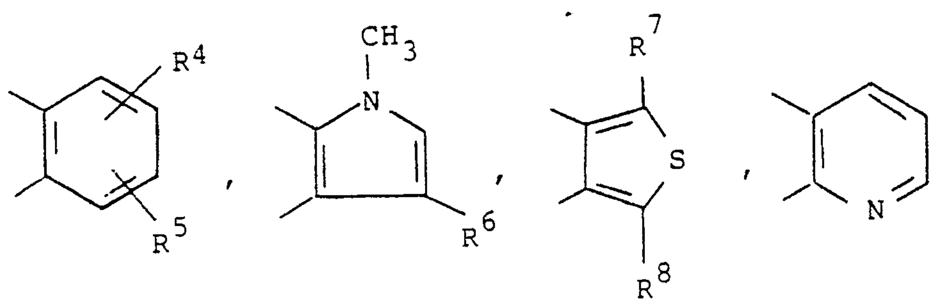

(S) (T) (U) (V)

darstellt und

X, R, $R^4$, $R^5$, $R^6$, $R^7$, $R^8$, m, n, o, p, $A^1$, $A^2$, $A^3$ und $A^4$ die folgenden Bedeutungen besitzen:

X ist eine =CH-Gruppe oder ein Stickstoffatom,

R ist ein geradkettiger oder verzweigter Alkylrest mit 1 bis 4 Kohlenstoffatomen, der gegebenenfalls noch durch ein durch Chlor, Brom, Fluor, Methyl oder Methoxy mono- oder disubstituiertes Phenyl substituiert sein kann,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 0, 1, 2 oder 3 mit folgenden Einschränkungen:

die Summe aus m+n und die Summe aus o+p bedeutet jeweils die Zahlen 1, 2 oder 3,

die Summe aus n+o und die Summe aus m+p bedeutet jeweils die Zahlen 1, 2, 3, 4 oder 5,

wobei jedoch die Summe aus m + n + o + p immer größer als 2 sein muß,
$A^1$, $A^2$, $A^3$, $A^4$ bedeuten Wasserstoffatome; für den Fall, daß m, n, o und p jeweils die Zahl 1 bedeuten, können entweder $A^1$ und $A^2$ zusammen oder $A^3$ und $A^4$ zusammen auch eine Ethylenbrücke darstellen,
ihrer Isomeren und ihrer Salze mit anorganischen oder organischen Säuren, dadurch gekennzeichnet, daß
a.) zur Herstellung basisch substituierter kondensierter Diazepinone der allgemeinen Formel Ia

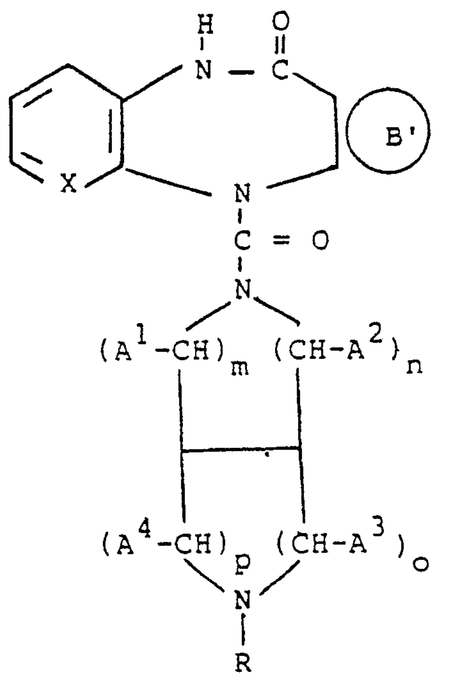

(Ia)

in der
X, R, $R^4$, $R^5$, $R^6$, $R^7$, $R^8$, m, n, o, p, $A^1$, $A^2$, $A^3$, $A^4$ die oben angegebenen Bedeutungen haben und

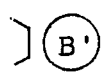

einen der zweiwertigen Reste (S), (U), (V) oder (T′)

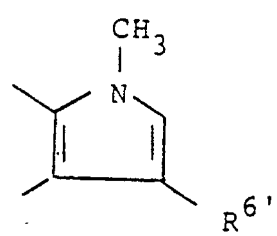

(T′)

darstellt worin $R^4$, $R^5$, $R^7$ und $R^8$ wie oben definiert sind und
$R^{6'}$ ein Chloratom oder eine Methylgruppe bedeutet, ein Kohlensäurederivat der allgemeinen Formel II

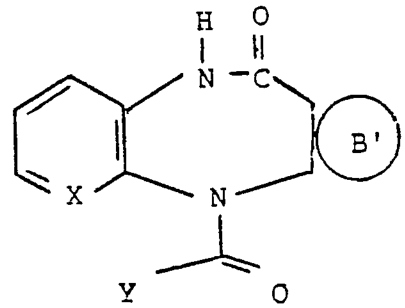

(II)

in der

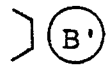

und X wie oben definiert sind und
Y ein Halogenatom oder den Rest bedeutet, in dem $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit einer Verbindung der allgemeinen Formel III

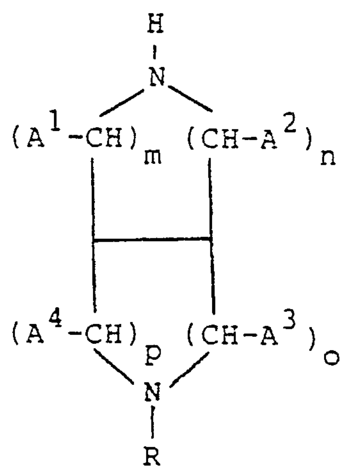

(III)

oder mit ihrer Metallvervindung der allgemeinen Formel IIIa

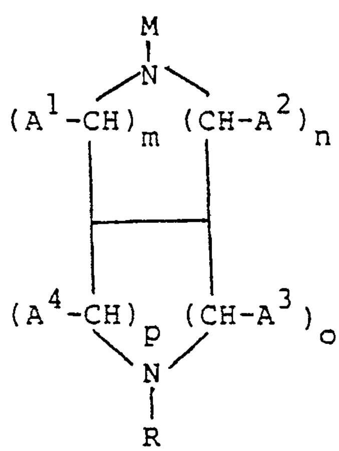

(IIIa)

in welchen R, m, n, o, p, $A^1$, $A^2$, $A^3$, $A^4$ wie oben definiert sind und M ein Alkalimetallatom oder 1 Äquivalent

eines Erdalkalimetallatoms bedeutet, gegebenennfalls in Anwesenheit eines Lösungsmittels, bei Temperaturen zwischen -10°C und dem Siedepunkt des Reaktionsgemisches, gegebenenfalls in Gegenwart einer Base oder eines Überschußes der Verbindung der allgemeinen Formel III, umgesetzt wird, oder

b.) zur Herstellung basisch substituierter kondensierter Diazepinone der vorgenannten allgemeinen Formel Ia

ein Tricyclus der allgemeinen Formel IV

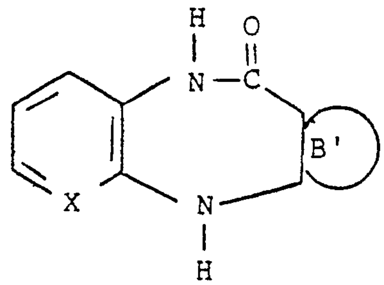

(IV)

in der

X und

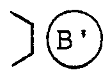

wie oben definiert sind, mit einem Chlorkohlensäurederivat der allgemeinen Formel V

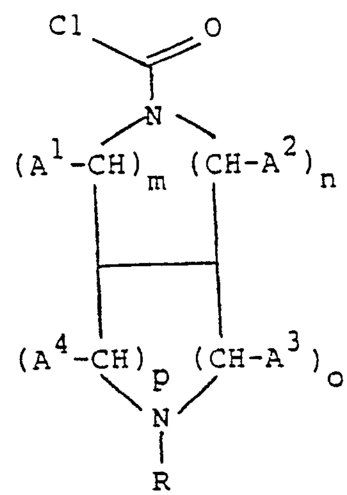

(V)

in der

R, m, n, o und p, $A^1$, $A^2$, $A^3$ und $A^4$ die oben erwähnten Bedeutungen haben,

in inerten Lösungsmitteln, vorzugsweise in Gegenwart von Basen, bei Temperaturen zwischen 30 und 100°C umgesetzt werden, oder

c.) zur Herstellung von unter die allgemeine Formel I fallenden Pyrrolo-kondensierten Diazepinonen der allgemeinen Formel Ib

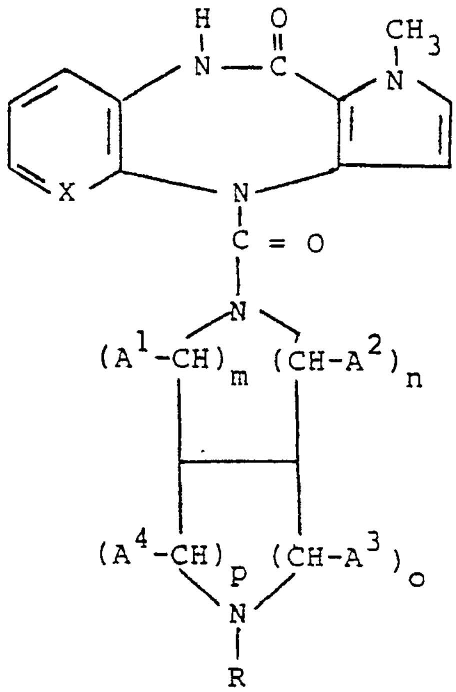

(Ib)

in der
X, R, m, n, o, p, $A^1$, $A^2$, $A^3$ und $A^4$ die oben erwähnten Bedeutungen besitzen, Verbindungen der allgemeinen Formel Ia, in der

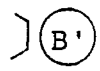

den zweiwertigen Rest (T′)

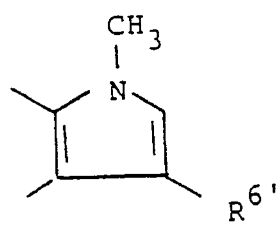

(T')

bedeutet, worin $R^{6'}$ ein Chloratom darstellt, einer Hydrogenolyse unterworfen werden, und, gegebenenfalls anschließend, so erhaltene Verbindungen der allgemeinen Formel I in ihre Isomeren aufgetrennt und/oder mit anorganischen oder organischen Säuren in ihre Salze übergeführt werden.

2.) Verfahren gemäß Anspruch 1c, dadurch gekennzeichnet, daß die Hydrogenolyse in Gegenwart von Katalysatoren von Metallen der VIII. Nebengruppe des Periodensystem, bei Wasserstoffdrucken von 1 bis 300 bar und Temperaturen von 0° bis 130° C in Gegenwart von Lösungsmitteln durchgeführt wird.

3.) Verfahren gemäß Anspruch 1c, dadurch gekennzeichnet, daß die Hydrogenolyse

a.) mit Ameisensäure und einem Palladium-auf-Kohle-Katalysator bei Temperaturen zwischen 70 und 110° C in Gegenwart eines Lösungsmittels oder

b.) mit Triethylammoniumformiat in Gegenwart überschüssigen Triethylamins und von Palladium auf Tierkohle oder

c.) mit Palladiumacetat und Triarylphosphinen bei Temperaturen zwischen 40 und 110° C erfolgt.